# EUROPÄISCHE PATENTANMELDUNG

(11) **EP 4 617 732 A1**
(43) Veröffentlichungstag der Anmeldung: **17.09.2025**
(21) Anmeldenummer: 24163527.5
(22) Anmeldetag: 14.03.2024
(51) Int. Cl.: G01T 1/172, G01T 1/24, G01T 1/29

(54) **PHOTONENZÄHLENDER RÖNTGENDETEKTOR, VERFAHREN ZUM BETREIBEN EINES PHOTONENZÄHLENDEN RÖNTGENDETEKTORS UND RÖNTGENGERÄT**

(71) Anmelder: Siemens Healthineers AG, 91301 Forchheim (DE)
(72) Erfinder: Göderer, Edgar, 91301 Forchheim (DE); Hosemann, Michael, 91056 Erlangen (DE); Hupfer, Martin, 91052 Erlangen (DE); Kreisler, Björn, 91353 Hausen (DE); Reitz, Bodo, 91301 Forchheim (DE)
(74) Vertreter: Siemens Healthineers Patent Attorneys

(57) **Zusammenfassung**

Die Erfindung betrifft einen photonenzählenden Röntgendetektor, umfassend mehrere Pixelelemente und ein Konverterelement zur Umwandlung von Röntgenstrahlung in elektrische Signale,
wobei in einer ersten Signalverarbeitungsstufe die Pixelelemente jeweils einen Komparator und eine Monoflop-Einheit mit einer Verzögerungseinheit aufweisen,
wobei der Komparator ausgebildet ist, das elektrische Signal mit einem Signalschwellwert zu vergleichen und ein digitales Pixelsignal an die Monoflop-Einheit bereitzustellen,
wobei die Monoflop-Einheit ausgebildet ist, anhand des digitalen Pixelsignals ein Pulssignal mit definierter Pulslänge bereitzustellen,
wobei in einer zweiten Signalverarbeitungsstufe Ausgänge der ersten Signalverarbeitungsstufe mit jeweils einer Verzögerungseinheit signaltechnisch gekoppelt sind, welche dazu ausgebildet ist, die Pulssignale mit einer jeweils angepassten Verzögerung an jeweils ein Zählelement bereitzustellen,
wobei das jeweilige Zählelement dazu ausgebildet ist, ein Zählsignal in Abhängigkeit des jeweiligen angepassten Pulssignals zu zählen.

Die Erfindung betrifft weiterhin ein Verfahren zum Betreiben eines photonenzählenden Röntgendetektors und ein Röntgengerät.

## Beschreibung

Die vorliegende Erfindung betrifft einen photonenzählenden Röntgendetektor, ein Verfahren zum Betreiben eines photonenzählenden Röntgendetektors und ein Röntgengerät.

Photonenzählende Röntgendetektoren finden in vielen bildgebenden Anwendungen Einsatz. So werden diese Röntgendetektoren beispielsweise in Computertomographie-Geräten (CT-Geräte) in der medizinischen Bildgebung genutzt, um ein tomographisches Röntgenbild eines Untersuchungsbereiches eines Untersuchungsobjekts, beispielsweise einer Patientin oder eines Patienten, zu erzeugen.

Als photonenzählendender Röntgendetektor kann insbesondere ein photonenzählendender, direkt-konvertierender Röntgendetektor eingesetzt werden. Eintreffende Röntgenstrahlung bzw. Photonen können in solchen Röntgendetektoren durch ein geeignetes Konvertermaterial in elektrische Signale umgewandelt werden. Als Konvertermaterial können beispielsweise CdTe, CdZnTe, CdTeSe, CdZnTeSe, CdMnTe, GaAs, Si, Ge oder andere verwendet werden. Hierbei können Röntgenphotonen, insbesondere Röntgenquanten, im direkt-konvertierenden Konvertermaterial absorbiert werden. Dabei können eine oder mehrere Ladungswolken erzeugt werden. Wenn es im Rahmen des Absorptionsprozesses zu einer Emission von Fluoreszenzphotonen kommt, werden diese meist nach einer typischen Absorptionslänge, beispielsweise ca. 100 um in CdTe oder CdZnTe, wieder absorbiert und erzeugen dort eine weitere Ladungsdeposition. Die Ladungswolken werden durch ein elektrisches Feld im Konvertermaterial getrennt und influenzieren auf ihrem Weg zu Pixelelektroden von Pixelelementen des Röntgendetektors ein elektrisches Signal in den Pixelelektroden. Ein zeitlicher Verlauf des elektrischen Signals auf den Pixelelektroden der Pixelelemente hängt dabei von einer räumlichen Position der bewegten Ladungen ab. Typischerweise wird nicht nur auf einer zentral unter einem Absorptionspunkt des Röntgenquants liegenden Pixelelektrode, sondern gleichzeitig auch auf den benachbarten Pixelelektroden ein elektrisches Signal influenziert. Das influenzierte elektrische Signal, insbesondere ein Signalpuls, kann in jedem der Pixelelemente verstärkt und typischerweise mit einer Komparatorschwelle verglichen werden. Überschreitet der Signalpuls die Komparatorschwelle, kann ein Zählereignis ausgelöst werden. Je nach Position und Größe der Ladungswolken kann das influenzierte elektrische Signal daher auch in den benachbarten Pixelelementen eines jeweiligen Pixelelements ein Zählereignis auslösen, selbst wenn der Anteil der dort deponierten Ladung geringer ist. Diese Zählereignisse finden, insbesondere auf einer Zeitskala von einigen Nanosekunden, gleichzeitig statt, daher nennt man diese koinzidierenden Zählereignisse auch Koinzidenz-Counts.

Ein häufig verbreiteter Umgang mit dem Problem koinzidierender Zählereignisse ist die normale Registrierung der Zählereignisse, als ob es reguläre Röntgenphotonen wären. Hierbei werden daher nicht die elektrischen Signale eines Röntgenspektrums, sondern konkreter die elektrischen Signale eines Pulshöhenspektrums erfasst. Eine Ladungsteilung (engl. Charge-Sharing) mit den zu dem jeweiligen Pixelelement benachbarten Pixelelementen erzeugt dabei ein "Low-Energy-Tailing", das heißt eine erhöhte, zusätzliche Zählrate bei niedrigeren Energien, die nicht durch niederenergetische Röntgenphotonen entsteht. Ferner verursachen die Fluoreszenzvorgänge oftmals zusätzliche Zählereignisse bei Fluoreszenzenergien sowie eine korrespondierende Unterschätzung einer Energie von betroffenen Absorptionsereignissen höherer Energie (engl. "K-Escape"). Häufig fehlt hierbei die Energie des Fluoreszenzphotons und das primäre Zählereignis hat entsprechend weniger Signalhöhe. Insgesamt entsteht somit eine nachteilige Verunreinigung des Röntgenspektrums und eine Erhöhung eines Rauschens durch die zusätzlichen Zählereignisse. Dies bedingt eine schlechtere spektrale Trennfähigkeit, insbesondere eine Mehr- oder Dualenergieauflösung, sowie ein suboptimales Signal-zu-Rauschen-Verhältnis (engl. signal-to-noise ratio, SNR), ein suboptimales Kontrast-zu-Rauschen-Verhältnis (engl. contrast-to-noise ratio, CNR) und/oder eine suboptimale detektive Quanteneffizienz (engl. detective quantum efficiency, DQE).

Eine geläufige Gegenmaßnahme ist die analoge Ladungssummation (engl. charge summing). Hierbei werden die gleichzeitig deponierten elektrischen Signale benachbarter Pixelelemente aufsummiert und beispielsweise dem meist-beitragenden Pixelelement zugeordnet. Nachteil dieser Lösung ist neben dem technischen Aufwand, insbesondere dem damit verbundenen Raum- und Energiebedarf, typischerweise eine deutliche Verlängerung einer Pulsbreite und/oder Totzeit des Röntgendetektors um einen Faktor zwischen 10 und 100. Die Zeit, bis das nächste Röntgenphoton wieder ohne Störung durch das vorherige Zählereignis nachgewiesen werden kann, verlängert sich also deutlich. Damit reduziert sich reziprok ein Röntgenfluss, bei dem die spektrale Bildgebung ohne übermäßige Degradation durch Überlagerung aufeinanderfolgender Signalpulse (engl. pile-up) genutzt werden kann. Dadurch sind die analogen Charge-Summing-Lösungen vor allem für niedrige Röntgenphotonenflüsse und nicht für typischen Flüsse in einem CT geeignet.

Eine weitere Möglichkeit besteht in einer Unterdrückungslogik für Koinzidenzereignisse (engl. anti-coincidence logic oder coincidence inhibition logic). Hierbei werden Zählereignisse blockiert, die gleichzeitig in benachbarten Pixelelementen registriert werden. Beispielsweise kann ein erstes Pixelelement, welches einen elektrischen Signalpuls bemerkt, das Zählen in benachbarten Pixelelementen unterdrücken, bis es mit der Verarbeitung des Signalpulses fertig ist. Alternativ oder zusätzlich kann auch sein Zählereignis unterdrückt werden, wenn während der Verarbeitung Zählereignisse in den benachbarten Pixelelementen identifiziert werden.

Ein Beispiel hierfür ist in der Druckschrift WO 2017/032548 A1 genannt. Nachteil einer solchen Unterdrückung ist häufig, dass eine Anzahl tatsächlich registrierter Zählereignisse sinkt. Insbesondere bei hohem Signalfluss, hohen Totzeiten, Pulsbreiten und/oder Shapingzeiten und großen Pixelgrößen kann es hierbei zu einem nicht mehr vernachlässigbaren, insbesondere vollständigen, Verlust des Nutzsignales kommen.

Es ist daher die Aufgabe der vorliegenden Erfindung, eine Möglichkeit anzugeben, das Erzeugen von Röntgenbilddatensätzen, insbesondere unter Berücksichtigung von auftretenden Koinzidenzen, weiter zu verbessern.

Die Aufgabe wird erfindungsgemäß gelöst durch den Gegenstand der unabhängigen Ansprüche. Vorteilhafte Ausführungsformen mit zweckmäßigen Weiterbildungen sind Gegenstand der Unteransprüche. Unabhängig vom grammatikalischen Geschlecht eines bestimmten Begriffes sind Personen mit männlicher, weiblicher oder anderer Geschlechteridentität mit umfasst.

Die Erfindung betrifft in einem ersten Aspekt einen photonenzählenden Röntgendetektor, welcher mehrere Pixelelemente und ein Konverterelement zur Umwandlung von Röntgenstrahlung in elektrische Signale umfasst. In einer ersten Signalverarbeitungsstufe weisen die Pixelelemente der mehreren Pixelelemente jeweils zumindest einen Komparator und die mehreren Pixelelemente zumindest eine Monoflop-Einheit auf. Der zumindest eine Komparator ist dazu ausgebildet, das elektrische Signal mit einer Energieschwelle zu vergleichen und ein digitales Pixelsignal in Abhängigkeit des Vergleichs an die zumindest eine Monoflop-Einheit bereitzustellen. Die zumindest eine Monoflop-Einheit ist dazu ausgebildet, basierend auf dem digitalen Pixelsignal ein Pulssignal mit definierter Pulslänge an einen Ausgang der ersten Signalverarbeitungsstufe bereitzustellen. In einer zweiten Signalverarbeitung sind die Ausgänge der ersten Signalverarbeitung der mehreren Pixelelementen mit jeweils einer Verzögerungseinheit signaltechnisch gekoppelt. Die jeweilige Verzögerungseinheit ist dazu ausgebildet, die Pulssignale mit einer jeweils angepassten Verzögerung als angepasste Pulssignale an jeweils ein Zählelement bereitzustellen. Dabei basiert die jeweils angepasste Verzögerung auf einer schaltungsbedingten Verzögerung des jeweils zumindest einen Pixelelements der mehreren Pixelelemente. Das jeweilige Zählelement ist dazu ausgebildet, ein Zählsignal in Abhängigkeit des jeweiligen zumindest einen angepassten Pulssignals zu zählen.

Der im Rahmen der Erfindung verwendete photonenzählende Röntgendetektor kann auch als direkt-konvertierender Röntgendetektor bezeichnet werden. Direkt konvertierende Röntgendetektoren werden meist in einem Stapelaufbau realisiert, bei dem an eine Lage des Konvertermaterials, das heißt an das Konverterelement, unterseitig eine zugeordnete Auswerteeinheit angebunden ist. Das Konverterelement kann insbesondere ein Konvertermaterial zum Umzuwandeln eingehender Röntgenstrahlung in das elektrische Signal umfassen. Das Konvertermaterial kann beispielsweise CdTe, CdZnTe, CdTeSe, CdZnTeSe, CdMnTe, GaAs, Si, Ge oder ein anderes geeignetes Material sein. Besonders vorteilhaft kann die Verwendung von CdTe sein. Die Unterseite des Konverterelements weist üblicherweise matrixförmig eine Mehrzahl von Elektroden, im Folgenden auch Sensor-Pixelelektroden genannt, in Form von metallisierten Kontaktelementen auf. Mit diesen ist die Auswerteeinheit signal-übertragungstechnisch kontaktiert, beispielsweise verlötet. Üblicherweise steht dabei einem konverterseitigen Kontaktelement jeweils ein pixelförmig ausgeführtes Gegenkontaktelement, im Folgenden auch Pixelelektrode genannt, auf Seiten der Auswerteeinheit entgegen. Die Auswerteeinheit stellt dann üblicherweise pixelweise Pixelelektroniken für die pixelweise Verarbeitung eines über die Pixelelektroden eingegangenen Signals bereit. Eintreffende Röntgenstrahlung wird im Konvertermaterial des Konverterelements in Abhängigkeit der lokal deponierten Energie eines Röntgenphotons in Ladungsträger konvertiert, basierend auf welchen in der pixelweise Pixelelektronik ein Signal, üblicherweise ein elektrischer Puls, erzeugt wird, welcher weiterverarbeitet wird. Einer pixelweisen Pixelelektronik kann ein entsprechendes Detektionsvolumen im Konverterelement zugeordnet werden, welches im Wesentlichen durch das elektrische Feld zwischen einer jeweiligen Sensor-Pixelelektrode und einer Top-Elektrode, welche auf der gegenüberliegenden Seite des Konverterelements aufgebracht ist, ausgebildet ist und welches das sensitiven Detektionsvolumen eines Pixelelements bildet.

Erfindungsgemäß weist jedes Pixelelement der mehreren Pixelelemente des erfindungsgemäßen photonenzählenden Röntgendetektors eine erste, pixelweise Signalverarbeitungsstufe auf. Die Pixelelemente der mehreren Pixelelemente weisen in der ersten Signalverarbeitungsstufe jeweils zumindest einen Komparator, insbesondere mehrere Komparatoren, auf. Ferner weisen die Pixelelemente der mehreren Pixelelemente in der ersten Signalverarbeitungsstufe zumindest eine Monoflop-Einheit auf. Dabei können die Pixelelemente der mehreren Pixelelemente in der ersten Signalverarbeitungsstufe jeweils zumindest eine Monoflop-Einheit aufweisen. Alternativ können ein oder mehrere Pixelelemente der mehreren Pixelelemente in der ersten Signalverarbeitungsstufe zumindest eine, insbesondere gemeinsame oder separate, Monoflop-Einheit aufweisen.

Der jeweilige zumindest eine Komparator kann dazu ausgebildet sein, das von dem Konverterelement in einem Betriebszustand des Röntgendetektors bereitgestellte elektrische Signal mit einem Signalschwellwert zu vergleichen. Der Röntgendetektor kann vorteilhafterweise eine analoge Signalformungskette aufweisen, welche dazu ausgebildet sein kann, das von dem Konverterelement bereitgestellte elektrische Signal zu formen und/oder zu verstärken und an den zumindest einen Komparator bereitzustellen. Der Signalschwellwert kann dabei eine Energieschwelle charakterisieren. Der Signalschwellwert kann dazu ausgebildet sein, eine Größe, beispielsweise Amplitude, des elektrischen Signals zu erfassen. Bei einem Überschreiten des Signalschwellwerts durch das elektrische Signal kann der jeweilige zumindest eine Komparator das digitale Pixelsignal in dem Betriebszustand des Röntgendetektors an die zumindest eine Monoflop-Einheit bereitstellen. Der Begriff "Komparator" ist im Rahmen dieser Erfindung breit aufzufassen. Insbesondere ist damit allgemein ein Vergleichsglied gemeint, welches ein Ausgangssignal auslöst, wenn der eingestellte Signalschwellwert durch das eingehende elektrische Signal überschritten wird. Der jeweilige Signalschwellwert des zumindest einen Komparators kann fest eingestellt sein. Beispielsweise kann der jeweilige Signalschwellwert fest durch die Herstellung des zumindest einen Komparators vorgegeben sein. Alternativ kann der jeweilige Signalschwellwert des zumindest einen Komparators veränderbar bzw. einstellbar sein. Beispielsweise kann der jeweilige Signalschwellwert auf eine Mehrzahl an festen Signalschwellwerten einstellbar sein. Alternativ oder zusätzlich kann der jeweilige Signalschwellwert innerhalb eines Signalschwellwertbereichs einstellbar sein. Der zumindest eine Komparator kann somit dazu ausgebildet sein, mittels des Vergleichs das jeweilige elektrische Signal in jeweils einen Energiebereich, insbesondere ein Energiefenster, einzusortieren.

Weist die erste Signalverarbeitungsstufe mehrere Komparatoren auf, so können die mehreren Komparatoren dazu ausgebildet sein, jeweils ein digitales Pixelsignal an eine gemeinsame Monoflop-Einheit oder an jeweils eine Monoflop-Einheit bereitzustellen. An dem zumindest einen Komparator kann im Wesentlichen ein Übergang von dem elektrischen, insbesondere analogen, Signal zu zumindest einem digitalen Signal, insbesondere dem zumindest einen digitalen Pixelsignal, erfolgen.

Die erste Signalverarbeitungsstufe eines Pixelelements der mehreren Pixelelemente kann auch mehr als einen Komparator mit jeweils einem Signalschwellwert aufweisen, so dass mehrere digitale Pixelsignale in Abhängigkeit der jeweiligen Signalschwellwerte bereitstellbar sind. Dabei können die Komparatoren des zumindest einen Pixelelements der mehreren Pixelelemente vorteilhafterweise verschiedene Signalschwellwerte aufweisen, welche verschiedene Energieschwellen charakterisieren.

Die zumindest eine Monoflop-Einheit kann eine monostabile Kippstufe, einen monostabilen Multivibrator und/oder Univibrator umfassen. Die zumindest eine Monoflop-Einheit kann als digitale Schaltung ausgebildet sein, welche genau einen stabilen Zustand aufweist. Ferner kann die zumindest eine Monoflop-Einheit dazu ausgebildet sein, für eine vordefinierte Zeitdauer durch ein eingehendes Signal, insbesondere das digitale Pixelsignal, in den stabilen Zustand getriggert zu werden. Nach der vordefinierten Zeitdauer kann die zumindest eine Monoflop-Einheit in den Ruhezustand zurückkehren. Vorteilhafterweise kann die zumindest eine Monoflop-Einheit dazu ausgebildet sein, durch eine Änderung des digitalen Pixelsignals, beispielsweise eine steigende Flanke des digitalen Pixelsignals, in den stabilen Zustand getriggert zu werden. Die zumindest eine Monoflop-Einheit kann ferner dazu ausgebildet sein, in dem stabilen Zustand das Pulssignal mit der definierten Pulslänge bereitzustellen. Hierfür kann die Monoflop-Einheit beispielsweise einen Pulssignalgenerator umfassen. Die definierte Pulslänge kann der vordefinierten Zeitdauer der zumindest einen Monoflop-Einheit entsprechen. Vorteilhafterweise kann mittels der zumindest einen Monoflop-Einheit das Pulssignal mit normalisierter Pulsbreite bereitstellbar sein. Insbesondere kann das durch die zumindest eine Monoflop-Einheit bereitstellbare jeweilige Pulssignal als längennormalisiertes Pulssignal aufgefasst werden.

Vorteilhafterweise sind die Ausgänge der ersten Signalverarbeitungsstufe der mehreren Pixelelemente in der zweiten Signalverarbeitungsstufe mit jeweils einer Verzögerungseinheit signaltechnisch gekoppelt, insbesondere verbunden. Die signaltechnische Kopplung kann eine Weiterleitung der an den Ausgängen der ersten Signalverarbeitungsstufe bereitgestellten Pulssignale an die Verzögerungseinheiten der zweiten Signalverarbeitungsstufe ermöglichen.

Vorteilhafterweise kann die zweite Signalverarbeitungsstufe zumindest zu dem jeweiligen Pixelelement, insbesondere zu jedem Ausgang der ersten Signalverarbeitungsstufe, jeweils eine Verzögerungseinheit aufweisen. Die zumindest eine Verzögerungseinheit kann eine Verzögerungsschaltung umfassen, welche dazu ausgebildet ist, das jeweilige Pulssignal mit der jeweiligen Verzögerung als angepasstes Pulssignal an das jeweilige Zählelement bereitzustellen. Die zumindest eine Verzögerungseinheit kann beispielsweise zur digitalen Signalanpassung und/ oder zur signaltechnischen, insbesondere analogen und/ oder digitalen, Verzögerung des jeweiligen Signals, insbesondere des jeweiligen Pulssignals, ausgebildet sein. Somit kann die zweite Signalverarbeitungsstufe zu dem jeweiligen Pixelelement, insbesondere zu jedem der mehreren Pixelelemente, jeweils zumindest eine Verzögerungseinheit aufweisen.

Die jeweilige, insbesondere pixelweise, angepasste Verzögerung kann auf einer schaltungsbedingten, insbesondere leitungsbedingten, Verzögerung des jeweils zumindest einen Pixelelements der mehreren Pixelelemente basieren, welches der jeweiligen Verzögerungseinheit zugeordnet ist. Insbesondere kann die jeweilige angepasste Verzögerung auf einer Differenz eines Höchstwerts einer schaltungsbedingten, insbesondere leitungsbedingten, Verzögerung von Pixelelementen einer Gruppe von Pixelelementen der mehreren Pixelelemente und der schaltungsbedingten, insbesondere leitungsbedingten, Verzögerung des jeweiligen zumindest einen Pixelelements der Gruppe von Pixelelementen der mehreren Pixelelemente, welches der Verzögerungseinheit zugeordnet ist, basieren. Hierdurch können die Pulssignale zumindest der Gruppe von Pixelelementen der mehreren Pixelelemente laufzeitkorrigiert an das jeweilige Zählelement bereitstellbar sein. Gemäß einer Ausführungsform kann die Gruppe von Pixelelementen jedes der mehreren Pixelelemente umfassen, sodass die Pulssignale aller Pixelelemente der mehreren Pixelelemente laufzeitkorrigiert an das jeweilige Zählelement bereitstellbar sind.

Die angepassten Pulssignale der mehreren Pixelelemente können von den Verzögerungseinheiten an jeweils ein Zählelement der zweiten Signalverarbeitungsstufe bereitstellbar sein. Alternativ können die angepassten Pulssignale der mehreren Pixelelemente an jeweils mehrere Zählelemente bereitstellbar sein. Insbesondere können die angepassten Pulssignale jeweils eines der Pixelelemente der mehreren Pixelelemente an dedizierte Zählelemente bereitstellbar sein. Alternativ können die angepassten Pulssignale einer Gruppe von Pixelelementen der mehreren Pixelelemente an zumindest ein gemeinsames Zählelement bereitstellbar sein.

Die Zählelemente können jeweils dazu ausgebildet sein, ein Zählsignal in Abhängigkeit des jeweiligen zumindest einen angepassten Pulssignals, insbesondere der jeweiligen mehreren angepassten Pulssignale, zu zählen. Dabei kann das jeweilige Zählsignal beispielsweise ein Pixel-Zählsignal oder ein Koinzidenz-Zählsignal umfassen. Die Zählelemente können jeweils dazu ausgebildet sein, basierend auf dem in einem Betriebszustand des Röntgendetektors eingehenden zumindest einen angepassten Pulssignals einen Zählerstand eines Zählers um eine Zähleinheit zu erhöhen, insbesondere bei Eingang des zumindest einen angepassten Pulssignals innerhalb eines vorgegebenen Zeitabschnitts. Die Zählelemente können jeweils eine Mehrzahl an Zählern umfassen. Die Zählelemente können beispielsweise einem Ressourcenblock aufweisend eine Mehrzahl an Zählern entsprechen, so dass basierend auf mehreren Signalausgängen jeweils eine Anzahl an bereitgestellten angepassten Pulssignalen gezählt und zumindest zeitweise gespeichert werden kann. Die Zählelemente können jeweils dazu ausgebildet sein, das Zählsignal aufweisend eine Information zu dem jeweiligen Zählerstand des jeweils zumindest einen Zählers bereitzustellen.

Das Bereitstellen der Zählsignale kann ein Speichern auf einem computerlesbaren Speichermedium und/oder ein Anzeigen auf einer Darstellungseinheit und/oder ein Übertragen an eine Bereitstellungseinheit umfassen. Insbesondere kann eine graphische Darstellung der Zählsignale, insbesondere eine Rekonstruktion eines Röntgenbilddatensatzes basierend auf den Zählsignalen, mittels der Darstellungseinheit angezeigbar sein.

Der vorgeschlagene Röntgendetektor kann ein verbessertes Erzeugen von Röntgenbilddatensätzen, insbesondere unter Berücksichtigung von auftretenden Koinzidenzen, ermöglichen. Durch die Pulsbreitennormalisierung mittels der zumindest einen Monoflop-Einheit können beispielsweise eine übermäßige Degradation durch Überlagerung aufeinanderfolgender Signalpulse (engl. pile-up) verringert werden. Des Weiteren kann die Anpassung des Pulssignals mittels der zumindest einen Verzögerungseinheit eine laufzeitkorrigierte Bereitstellung der jeweils angepassten Pulssignale an die jeweiligen Zählelemente ermöglichen. Hierdurch kann eine verbesserte, insbesondere präzisere, Weiterverarbeitung der an den Ausgängen der ersten Signalverarbeitungsstufe bereitgestellten Signale in der zweiten Signalverarbeitungsstufe ermöglicht werden, insbesondere hinsichtlich einer Erfassung von Koinzidenzen.

In einer weiteren vorteilhaften Ausführungsform des vorgeschlagenen Röntgendetektors können die Pixelelemente in der ersten Signalverarbeitung ferner jeweils einen Signalverstärker aufweisen. Dabei kann der Signalverstärker dazu ausgebildet sein, das elektrische Signal zu verstärken und das verstärkte elektrische Signal an den zumindest einen Komparator bereitzustellen.

Vorteilhafterweise können die Signalverstärker jeweils einen Signaleingang und einen Signalausgang aufweisen. Das Konverterelement kann das elektrische Signal des jeweiligen Pixelelements der mehreren Pixelelemente in einem Betriebszustand des Röntgendetektors an den Signaleingang des jeweiligen Signalverstärkers bereitstellen. Die Signalverstärker können jeweils dazu ausgebildet sein, das jeweils eingehende elektrische Signal zu verstärken, insbesondere einen Signalwert, beispielsweise eine Signalamplitude, zu erhöhen und das verstärkte elektrische Signal an den jeweiligen Signalausgang bereitzustellen, insbesondere an den zumindest einen Komparator bereitzustellen. Ferner können die Signalverstärker zur Signalformung des eingehenden elektrischen Signals ausgebildet sein.

Die vorgeschlagene Ausführungsform kann eine verbesserte Signalverarbeitung des elektrischen Signals durch den zumindest einen Komparator, die zumindest eine Monoflop-Einheit ermöglichen.

In einer weiteren vorteilhaften Ausführungsform des vorgeschlagenen Röntgendetektors kann die zumindest eine Monoflop-Einheit dazu ausgebildet sein, das Pulssignal bei Identifikation einer steigenden Flanke des digitalen Pixelsignal bereitzustellen.

Das digitale Pixelsignal kann verschiedene Signalzustände aufweisen, welche in zeitlicher Abfolge auftreten können. Die verschiedenen Signalzustände können dabei einen Ruhezustand (engl. baseline), insbesondere bei Nichtüberschreitung des Signalschwellwerts in dem zumindest einen Komparator, und einen Aktivzustand, insbesondere bei Überschreitung des Signalschwellwerts in dem zumindest einen Komparator, umfassen. An Übergängen zwischen den verschiedenen Signalzuständen kann das Pixelsignal jeweils eine Flanke, insbesondere eine Signalflanke, aufweisen. An den Übergängen zwischen einem Ruhezustand und einem Aktivzustand des Pixelsignals kann das Pixelsignal eine steigende Flanke aufweisen. An den Übergängen zwischen einem Aktivzustand und einem Ruhezustand des Pixelsignals kann das Pixelsignal eine fallende Flanke aufweisen. Vorteilhafterweise kann die zumindest eine Monoflop-Einheit dazu ausgebildet sein, steigende Flanken in dem jeweiligen Pixelsignal zu identifizieren. Ferner kann die zumindest eine Monoflop-Einheit dazu ausgebildet sein, das Pulssignal bei Identifikation einer steigenden Flanke, insbesondere nur bei Identifikation einer steigenden Flanke, des digitalen Pixelsignals bereitzustellen. Insbesondere kann die Monoflop-Einheit dazu ausgebildet sein, von einer steigenden Flanke des digitalen Pixelsignals in den aktiven Zustand getriggert zu werden.

Die vorgeschlagene Ausführungsform kann eine zuverlässige Auslösung eines jeweiligen Pulssignals durch die jeweilige Monoflop-Einheit nach Umwandlung eines Röntgenphotons im Konvertermaterial ermöglichen.

In einer weiteren vorteilhaften Ausführungsform des vorgeschlagenen Röntgendetektors können die Pixelelemente in der ersten Signalverarbeitungsstufe jeweils mehrere Komparatoren und zu jedem Komparator jeweils eine Monoflop-Einheit aufweisen. Dabei können die Komparatoren jeweils eines der mehreren Pixelelementen dazu ausgebildet sein, das elektrische Signal mit verschiedenen Signalschwellen zu vergleichen und jeweils ein digitales Pixelsignal in Abhängigkeit des Vergleichs an jeweilige Monoflop-Einheit bereitzustellen. Die Monoflop-Einheiten können dazu ausgebildet sein, basierend auf dem jeweiligen digitalen Pixelsignal jeweils ein Pulssignal mit definierter Pulslänge an den jeweiligen Ausgang der ersten Signalverarbeitungsstufe bereitzustellen.

Vorteilhafterweise können die mehreren Pixelelemente in der ersten Signalverarbeitungsstufe jeweils mehrere Komparatoren, insbesondere jeweils eine gleiche oder verschiedene Anzahl von Komparatoren, und zu jedem der Komparatoren jeweils eine Monoflop-Einheit aufweisen. Die Komparatoren jeweils eines der mehreren Pixelelemente können vorteilhafterweise verschiedene Signalschwellwerte aufweisen, welche jeweils einer pixelindividuellen oder pixelkombinierenden, insbesondere koinzidierenden, Energieschwelle entsprechen. Die Komparatoren jeweils eines der mehreren Pixelelemente können jeweils dazu ausgebildet sein, das elektrische Signal mit ihrem jeweiligen Signalschwellwert zu vergleichen und jeweils ein digitales Pixelsignal in Abhängigkeit des Vergleichs an die jeweilige Monoflop-Einheit bereitzustellen. Die Komparatoren von verschiedenen Pixelelementen zumindest einer Gruppe von Pixelelementen der mehreren Pixelelemente können vorteilhafterweise paarweise die gleichen Signalschwellwerte aufweisen.

Die Monoflop-Einheiten können dazu ausgebildet sein, basierend auf dem jeweiligen digitalen Pixelsignal, insbesondere bei Identifikation einer steigenden Flanke des digitalen Pixelsignals, jeweils ein Pulssignal mit definierter Pulslänge an den jeweiligen Ausgang der ersten Signalverarbeitungsstufe bereitzustellen.

Die vorgeschlagene Ausführungsform kann vorteilhaft eine verbesserte energieauflösende Erzeugung von Röntgenbilddatensätzen, insbesondere unter Berücksichtigung von auftretenden Koinzidenzen, ermöglichen.

In einer weiteren vorteilhaften Ausführungsform des vorgeschlagenen Röntgendetektors können in der zweiten Signalverarbeitungsstufe die Verzögerungseinheiten zumindest einer Gruppe von Pixelelementen der mehreren Pixelelemente mit einer Koinzidenzlogik und jeweils einem Zählelement signaltechnisch gekoppelt sein. Dabei können die angepassten Pulssignale der zumindest einen Gruppe von Pixelelementen an die Koinzidenzlogik bereitstellbar sein. Die Koinzidenzlogik kann dazu ausgebildet sein, ein koinzidierendes Auftreten von angepassten Pulssignalen der mehreren Pixelelementen zu identifizieren. Die jeweiligen Zählelemente der zumindest einen Gruppe von Pixelelementen können dazu ausgebildet sein, ein Pixel-Zählsignal zu zählen, welches auf einem in den Pixelelementen der Gruppe von Pixelelementen direkt eingegangenen Signal basiert, oder bei Identifikation einer Koinzidenz durch die Koinzidenzlogik ein Koinzidenz-Zählsignal zu zählen, welches auf dem in dem jeweiligen Pixelelements direkt eingegangenen Signal und auf dem koinzidierend auftretenden Signal zumindest eines weiteren Pixelelements der mehreren Pixelelemente basiert.

Vorteilhafterweise können in der zweiten Signalverarbeitungsstufe die Verzögerungseinheiten zumindest einer Gruppe von Pixelelementen, insbesondere von zumindest zwei Pixelelementen, der mehreren Pixelelemente mit der Koinzidenzlogik und jeweils zumindest einem Zählelement, insbesondere jeweils mehreren Zählelementen, signaltechnisch gekoppelt, insbesondere verbunden, sein. Insbesondere können in der zweiten Signalverarbeitungsstufe die Verzögerungseinheiten mehrerer, insbesondere sich teilweise überschneidender, Gruppen von Pixelelementen, insbesondere von jeweils zumindest zwei Pixelelementen, der mehreren Pixelelemente mit jeweils einer Koinzidenzlogik und jeweils zumindest einem Zählelement, insbesondere jeweils mehreren Zählelementen, signaltechnisch gekoppelt, insbesondere verbunden, sein.

Vorteilhafterweise kann die zumindest eine Gruppe von Pixelelementen in der zweiten Signalverarbeitungsstufe zumindest ein erstes Zählelement, insbesondere mehrere erste Zählelemente, und zumindest ein zweites Zählelement, insbesondere mehrere zweite Zählelemente, aufweisen. Das zumindest eine erste Zählelement kann dazu ausgebildet sein, ein Pixel-Zählsignal zu zählen, welches auf einem in den Pixelelementen der Gruppe von Pixelelementen direkt eingegangenen Signal, insbesondere dem Pulssignal oder dem digitalen Pixelsignal, basiert. Das zumindest eine erste Zählelement kann direkt der jeweiligen Verzögerungseinheit des jeweiligen Pixelelements der zumindest einen Gruppe von Pixelelementen nachgeschaltet sein.

Das zumindest eine zweite Zählelement kann dazu ausgebildet sein, bei Identifikation einer Koinzidenz durch die Koinzidenzlogik ein Koinzidenz-Zählsignal zu zählen, welches auf dem in dem jeweiligen Pixelelement direkt eingegangenen Signal und auf dem koinzidierend auftretenden Signal zumindest eines weiteren Pixelelements der mehreren Pixelelemente basiert. Das zumindest eine zweite Zählelement kann direkt der Koinzidenzlogik nachgeschaltet sein.

Die Zählelemente der zweiten Signalverarbeitungsstufe der zumindest einen Gruppe von Pixelelementen können zwischen einem Zustand als erstes oder zweites Zählelement umschaltbar, insbesondere konfigurierbar, ausgebildet sein.

Die Koinzidenzlogik kann dazu ausgebildet sein, bei Auftreten von zumindest zwei koinzidierend auftretenden Signalen, insbesondere angepassten Pulssignalen, ein Ausgangsignal bereitzustellen, welches mittels eines mit der Koinzidenzlogik signaltechnisch gekoppelten zweiten Zählelements als Koinzidenz-Zählsignal zählbar ist. Die Koinzidenzlogik kann beispielsweise als, insbesondere logischer, Koinzidenzbaum und/oder, insbesondere logische, Koinzidenzschaltung ausgebildet sein. Dabei kann die Koinzidenzlogik direkt den Verzögerungseinheiten der zumindest einen Gruppe von Pixelelementen nachgeschaltet sein.

Die vorgeschlagene Ausführungsform kann eine verbesserte Erfassung von koinzidierenden Zählereignissen ermöglichen. Die bereitgestellten Koinzidenz-Zählsignale können vorteilhaft zur Korrektur von Pixel-Zählsignalen verwendet werden. Ferner kann die vorgeschlagene Ausführungsform insbesondere im Hochflussfall vorteilhaft sein, da die Koinzidenzen aufgrund der definierten Pulslänge des jeweiligen angepassten Pulssignals nur in einem Zeitfenster um das erstmalige Überschreiten erzeugbar sind.

In einer weiteren vorteilhaften Ausführungsform des vorgeschlagenen Röntgendetektors kann die zweite Signalverarbeitungsstufe zumindest ein erstes Schaltelement aufweisen, welches dazu ausgebildet ist, die Koinzidenzlogik zu aktivieren oder zu deaktivieren. Dabei können die Zählelemente der zumindest einen Gruppe von Pixelelementen bei deaktivierter Koinzidenzlogik das Pixel-Zählsignal zählen.

Das erste Schaltelement kann einen Signaleingang und zwei Signalausgänge aufweisen. Vorteilhafterweise können die Signale, insbesondere die Pulssignale oder die digitalen Pixelsignale, von den Ausgängen der ersten Signalverarbeitungsstufe der zumindest einen Gruppe von Pixelelementen an den Signaleingang des ersten Schaltelements bereitstellbar sein. Das erste Schaltelement kann dazu ausgebildet sein, die an seinem Signaleingang eingehenden Signale wahlweise über seinen ersten Signalausgang an die Koinzidenzlogik und über seinen zweiten Signalausgang an jeweils ein erstes Zählelement bereitzustellen oder nur über seinen zweiten Signalausgang an jeweils ein erstes Zählelement bereitzustellen. Das Umschalten des ersten Schaltelements kann beispielsweise über eine Konfiguration (engl. config bit) erfolgen. Das erste Schaltelement kann ferner eine Kombination von Demultiplexer und Multiplexer zur Aktivierung bzw. Deaktivierung der Koinzidenzlogik umfassen.

Die vorgeschlagene Ausführungsform kann eine verbesserte Testbarkeit der Schaltung ermöglichen. Hierdurch können beispielsweise fehlerhafte Schaltkreise bereits während einer Produktion des Röntgendetektors identifiziert und Veredelungsverluste minimiert werden. Hierdurch kann zudem das Erzeugen von Röntgenbilddatensätzen, insbesondere unter Berücksichtigung von auftretenden Koinzidenzen, weiter verbessert werden.

In einer weiteren vorteilhaften Ausführungsform des vorgeschlagenen Röntgendetektors kann die zumindest eine Gruppe von Pixelelementen zumindest eines der Pixelelemente und zumindest ein weiteres an das Pixelelement reihen- oder spaltenweise direkt angrenzendes Pixelelement umfassen.

Vorteilhafterweise können die mehreren Pixelelemente zumindest teilweise, insbesondere vollständig, reihen- und/oder spaltenförmig, insbesondere matrixförmig, zueinander angeordnet sein. Vorteilhafterweise kann die zumindest eine Gruppe von Pixelelementen zumindest eines der Pixelelemente der mehreren Pixelelemente und zumindest ein weiteres Pixelelement, insbesondere mehrere weitere Pixelelemente, umfassen, welches reihen- oder spaltenförmig direkt an das zumindest eine Pixelelement angrenzt, insbesondere direkt benachbart zu dem zumindest einen Pixelelement angeordnet ist. Vorteilhafterweise kann die zumindest eine Gruppe von Pixelelementen das zumindest eine Pixel und alle reihen- und spaltenförmig direkt daran angrenzenden, insbesondere direkt benachbarten, Pixelelemente umfassen.

Vorteilhafterweise können in der zweiten Signalverarbeitungsstufe die Verzögerungseinheiten mehrerer Gruppen von Pixelelementen der mehreren Pixelelemente mit jeweils einer Koinzidenzlogik und jeweils einem Zählelement signaltechnisch gekoppelt sein. Die mehreren Gruppen von Pixelelementen der mehreren Pixelelemente können zumindest teilweise übereinstimmende Pixelelemente als das zumindest eine Pixelelement oder das zumindest eine weitere Pixelelement umfassen. Dabei können sich die mehreren Gruppen von Pixelelementen insbesondere in dem zumindest einen Pixelelement oder wenigstens einem des zumindest einen weiteren Pixelelements unterscheiden. Beispielsweise kann das zumindest eine weitere Pixelelement einer ersten Gruppe von Pixelelementen das zumindest eine Pixelelement oder das zumindest eine weitere Pixelelement einer weiteren Gruppe von Pixelelementen sein. Ferner kann das zumindest eine Pixelelement einer ersten Gruppe von Pixelelementen das zumindest eine weitere Pixelelement einer weiteren Gruppe von Pixelelementen sein.

Die vorgeschlagene Ausführungsform kann eine zuverlässige Identifikation von koinzidierenden Zählereignissen ermöglichen.

In einer weiteren vorteilhaften Ausführungsform des vorgeschlagenen Röntgendetektors kann der Röntgendetektor weiterhin eine Taktsignalerzeugungseinheit aufweisen, welche dazu ausgebildet ist, ein definiertes Taktsignal an die erste und/oder zweiten Signalverarbeitungsstufe bereitzustellen. Dabei können die erste und/oder die zweite Signalverarbeitungsstufe dazu ausgebildet sein, ihre jeweiligen Komponenten, welche zur zeitbasierten Verarbeitung und/oder Bereitstellung der jeweiligen Signale ausgebildet sind, anhand des Taktsignals anzupassen.

Das Taktsignal kann beispielswiese für Kalibrationen, Totzeitmessungen und/oder zur Verhinderung einer Paralyse einer jeweiligen Schaltung der ersten und/oder zweiten Signalverarbeitungsstufe bei hohen Röntgenflüssen vorgesehen sein. Das Taktsignal kann beispielsweise als Clock-Signal bereitstellbar sein. Durch regelmäßiges Bereitstellen eines Clock-Signals kann in der jeweiligen Signalverarbeitungsstufe ein Signal an den entsprechenden Komponenten registriert werden und somit eine Nichtparalysierbarkeit erreicht werden. Das Taktsignal kann an die erste und/oder zweite Signalverarbeitungsstufe zumindest einer Gruppe von Pixelelementen der mehreren Pixelelemente, insbesondere an die erste und/oder zweite Signalverarbeitungsstufe der mehreren Pixelelemente, bereitgestellt werden. Die Taktsignalerzeugungseinheit kann mehrere Subtaktsignalerzeugungseinheiten aufweisen, welche dazu ausgebildet sind, jeweils ein, insbesondere dediziertes, Taktsignal an eine der Signalverarbeitungsstufen und/oder jeweils eine Komponente der Signalverarbeitungsstufen bereitzustellen. Das von der Taktsignalerzeugungseinheit bereitstellbare Taktsignal kann beispielsweise für mehrere, insbesondere alle Komponenten, gleich, verschieden für verschiedene Signalschwellwerte, pixelindividuell oder komparatorindividuell bereitstellbar sein. Ferner kann die Taktsignalerzeugungseinheit zum Bereitstellen des zumindest einen Taktsignal durch eine steigende oder fallende Flanke des digitalen Pixelsignals triggerbar sein. Die Taktsignalerzeugungseinheit kann ferner dazu ausgebildet sein, eine Frequenz des bereitstellbaren Taktsignals an einen jeweiligen Signalschwellwert des zumindest einen Komparators anzupassen. Ferner kann die Taktsignalerzeugungseinheit dazu ausgebildet sein, das zumindest eine Taktsignal mit einer definierten Verzögerung bereitzustellen, beispielsweise dass ein erster Takt unterdrückt wird.

Die erste und/oder die zweite Signalverarbeitungsstufe der mehreren Pixelelemente können dazu ausgebildet sein, ihre jeweiligen Komponenten, insbesondere den zumindest einen Komparator und/oder die zumindest eine Monoflop-Einheit der ersten Signalverarbeitungsstufe und/oder die zumindest eine Verzögerungseinheit und/oder das zumindest eine Zählelement der zweiten Signalverarbeitungsstufe, anhand des Taktsignals anzupassen. Das Anpassen der jeweiligen Komponenten der ersten und/oder zweiten Signalverarbeitungsstufe basierend auf dem Taktsignal kann ein Kalibrieren und/oder Synchronisieren umfassen.

Die vorgeschlagene Ausführungsform kann vorteilhaft ein Kalibrieren und/oder Synchronisieren der jeweiligen Komponenten der ersten und/oder zweiten Signalverarbeitungsstufe basierend auf dem Taktsignal ermöglichen, insbesondere für ein oder mehrere, insbesondere alle, der mehreren Pixelelemente.

In einer weiteren vorteilhaften Ausführungsform des vorgeschlagenen Röntgendetektors kann die erste Signalverarbeitungsstufe zumindest ein zweites Schaltelement aufweisen, welches dazu ausgebildet ist, die zumindest eine Monoflop-Einheit zu aktivieren oder zu deaktivieren. Dabei kann der zumindest eine Komparator das digitale Pixelsignal bei deaktivierter Monoflop-Einheit an den jeweiligen Ausgang der ersten Signalverarbeitungsstufe bereitstellen.

Das zweite Schaltelement kann einen Signaleingang und zwei Signalausgänge aufweisen. Vorteilhafterweise kann der jeweilige zumindest eine Komparator das digitale Pixelsignal an den Signaleingang des zweiten Schaltelements bereitstellen. Das zweite Schaltelement kann dazu ausgebildet sein, das digitale Pixelsignal wahlweise über seinen ersten Signalausgang an die zumindest eine Monoflop-Einheit oder über seinen zweiten Signalausgang an den jeweiligen Ausgang der ersten Signalverarbeitungsstufe bereitzustellen. Das Umschalten des zweiten Schaltelements kann beispielsweise über eine Konfiguration (engl. config bit) erfolgen. Vorteilhafterweise können die mehreren Pixelelemente in der ersten Signalverarbeitungsstufe jeweils ein zweites Schaltelement, insbesondere jeweils ein zweites Schaltelement zu jedem Komparator des zumindest einen Komparators, aufweisen. Das zweite Schaltelement kann ferner eine Kombination von Demultiplexer und Multiplexer zur Aktivierung bzw. Deaktivierung der zumindest einen Monoflop-Einheit umfassen.

Die vorgeschlagene Ausführungsform kann ein optionales Deaktivieren der zumindest einen Monoflop-Einheit ermöglichen. Hierdurch kann vorteilhaft erreicht werden, dass ein für eine Registrierung von koinzidierenden Zählereignissen offenes Zeitfenster von einer deponierten Pulshöhe, insbesondere einer Dauer einer Überschreitung des jeweiligen Signalschwellwerts des jeweiligen Komparators, in dem jeweiligen Pixelelement abhängt. Hierdurch können die Pixelelemente, in denen bei einem Ereignis die meiste Ladung deponiert wird, ein längeres Koinzidenzfenster erhalten als Pixelelemente mit nur geringer Ladungsdeposition. Als Konsequenz kann das koinzidierende Zählereignis vorteilhafterweise mit höherer Wahrscheinlichkeit in den Pixelelementen mit der größten Ladungsdeposition registriert werden. Dies kann eine spektrale Ortsauflösung verbessern, da das Koinzidenz-Zählsignal somit klarer und korrekter, insbesondere bezüglich eines Ortes der primären Ladungsdeposition, lokalisiert ist.

Durch die Wahlmöglichkeit bezüglich der Nutzung des Monoflops kann die vorgeschlagene Schaltung vorteilhafterweise auf das zu erwartende digitale Pixelsignal angepasst werden. Im Niedrigflussfall kann eine erhöhte spektrale Ortsauflösung vorteilhaft sein. In Pile-Up-dominierten Messungen hingegen können eine durch die Monoflop-Einheit erreichbare Verkürzung eines auf Koinzidenz sensitiven Intervalls und/oder eine schnellere Wiederauslösbarkeit von Zählereignissen vorteilhafterweise zu einer Verringerung einer flussabhängigen Degradation des Koinzidenz-Zählsignals genutzt werden.

In einer weiteren vorteilhaften Ausführungsform des vorgeschlagenen Röntgendetektors kann die erste Signalverarbeitungsstufe zumindest ein drittes Schaltelement aufweisen, welches dazu ausgebildet ist, die Verzögerungseinheit zu aktivieren oder zu deaktivieren. Vorteilhafterweise kann bei Deaktivierung der zumindest einen Verzögerungseinheit das an die jeweilige Verzögerungseinheit der zumindest einen Verzögerungseinheit bereitgestellte Signal verzögerungsfrei an das jeweilige Zählelement bereitstellbar sein.

Das dritte Schaltelement kann einen Signaleingang und zwei Signalausgänge aufweisen. Vorteilhafterweise kann die jeweilige Monoflop-Einheit das Pulssignal an den Signaleingang des dritten Schaltelements bereitstellen. Weist die erste Signalverarbeitungsstufe zumindest ein zweites Schaltelement auf, kann in einem ersten Betriebszustand, insbesondere einem ersten Zustand des zweiten Schaltelements, die jeweilige Monoflop-Einheit das Pulssignal mittels des ersten Signalausgangs des zweiten Schaltelements an den Signaleingang des dritten Schaltelements bereitstellen. In einem zweiten Betriebszustand, insbesondere einem zweiten Zustand des zweiten Schaltelements, kann der jeweilige Komparator das digitale Pixelsignal mittels des zweiten Signalausgangs des zweiten Schaltelements an den Signaleingang des dritten Schaltelements bereitstellen. Das dritte Schaltelement kann dazu ausgebildet sein, das an seinem Signaleingang eingehende Signal, insbesondere das Pulssignal oder das digitale Pixelsignal, wahlweise über seinen ersten Signalausgang an die Verzögerungseinheit oder über seinen zweiten Signalausgang an das jeweilige Zählelement bereitzustellen. Das Umschalten des dritten Schaltelements kann beispielsweise über eine Konfiguration (engl. config bit) erfolgen. Vorteilhafterweise können die mehreren Pixelelemente in der ersten Signalverarbeitungsstufe jeweils ein drittes Schaltelement, insbesondere jeweils ein drittes Schaltelement zu jeder Verzögerungseinheit, aufweisen. Das zumindest eine dritte Schaltelement kann ferner eine Kombination von Demultiplexer und Multiplexer zur Aktivierung bzw. Deaktivierung der jeweiligen Verzögerungseinheit umfassen.

Die vorgeschlagene Ausführungsform kann eine verbesserte Testbarkeit der Schaltung ermöglichen. Hierdurch kann zudem das Erzeugen von Röntgenbilddatensätzen, insbesondere unter Berücksichtigung von auftretenden Koinzidenzen, weiter verbessert werden.

In einer weiteren vorteilhaften Ausführungsform des vorgeschlagenen Röntgendetektors können die definierte Pulslänge des durch die zumindest eine Monoflop-Einheit bereitstellbaren Pulssignals, insbesondere zwischen 1 ns und 20 ns, und/oder die Verzögerung des durch die zumindest eine Verzögerungseinheit bereitstellbaren angepassten Pulssignals anpassbar sein.

Vorteilhafterweise kann die definierte Pulslänge des durch die zumindest eine Monoflop-Einheit bereitstellbaren Pulssignals anpassbar, insbesondere einstellbar, sein. Insbesondere können die definierten Pulslängen der durch die mehreren Monoflop-Einheiten bereitstellbaren Pulssignale insgesamt oder individuell, insbesondere zumindest gruppenweise, anpassbar sein. Vorteilhafterweise kann die definierte Pulslänge des durch die zumindest eine Monoflop-Einheit bereitstellbaren Pulssignals auf einen Wert von 1 ns bis 20 ns anpassbar sein.

Alternativ oder zusätzlich kann die Verzögerung des durch die zumindest eine Verzögerungseinheit bereitstellbaren angepassten Pulssignals anpassbar, insbesondere einstellbar, sein. Insbesondere können die Verzögerungen der durch die mehreren Verzögerungseinheiten bereitstellbaren angepassten Pulssignale insgesamt oder individuell, insbesondere zumindest gruppenweise, anpassbar sein. Beispielsweise kann die zumindest eine Verzögerungseinheit das jeweilige Pulssignal mit einer zusätzlichen, insbesondere pixelunabhängigen, Verzögerung an das jeweilige Zählelement bereitstellen.

Der Röntgendetektor kann vorteilhafterweise ein Verstellelement, beispielsweise einen Schalter, und/oder eine Eingabeeinheit, beispielsweise eine Tastatur, aufweisen, welche dazu ausgebildet sind, die definierte Pulslänge des durch die zumindest eine Monoflop-Einheit bereitstellbaren Pulssignals und/oder die Verzögerung des durch die zumindest eine Verzögerungseinheit bereitstellbaren angepassten Pulssignals anzupassen, insbesondere zu erhöhen oder zu verringern.

Die Anpassbarkeit der definierten Pulslänge und Verzögerung kann vorteilhaft eine Verringerung von Laufzeitunterschieden zwischen verschiedenen Pixelelementen ermöglichen, so dass eine kürzere und damit vorteilhaftere definierte Pulslänge einstellbar ist. Bei festen definierten Pulslängen kann diese entweder zu kurz oder zu lang sein. Bei einer zu kurzen definierten Pulslänge kann es zum Verlust von koinzidierenden Zählereignissen kommen. Bei einer zu langen definierten Pulslänge kann eine Signalqualität im Falle höherer Flüsse suboptimal sein. Vorteilhafterweise kann die Anpassbarkeit der definierten Pulslänge eine Vermeidung dieser beiden Fälle, insbesondere einer zu kurzen oder zu langen definierten Pulslänge, ermöglichen.

Die Erfindung betrifft in einem zweiten Aspekt ein Verfahren zum Betreiben eines vorgeschlagenen photonenzählenden Röntgendetektors. In einem ersten Schritt a) wird Röntgenstrahlung mit dem photonenzählenden Röntgendetektor empfangen. Dabei wird die Röntgenstrahlung mittels des Konverterelements in elektrische Signale umgewandelt. In einem zweiten Schritt b) werden die erzeugten elektrischen Signale mittels der ersten Signalverarbeitungsstufe und der zweiten Signalverarbeitungsstufe des photonenzählenden Röntgendetektors verarbeitet. Dabei werden Pulssignale an die Ausgänge der ersten Signalverarbeitungsstufe bereitgestellt, welche mittels der zweiten Signalverarbeitungsstufe weiterverarbeitet werden. In einem dritten Schritt c) werden die Zählsignale der Zählelemente der zweiten Signalverarbeitungsstufe bereitgestellt.

Die Vorteile des Verfahrens entsprechen im Wesentlichen den Vorteilen des vorgeschlagenen photonenzählenden Röntgendetektors. Hierbei erwähnte Merkmale, Vorteile oder alternative Ausführungsformen können ebenso auch auf die anderen beanspruchten Gegenstände übertragen werden und umgekehrt.

Das Empfangen der Röntgenstrahlung in Schritt a) kann das Umwandeln der Röntgenstrahlung mittels des Konverterelements in elektrische Signale und das Bereitstellen der elektrischen Signale an den zumindest einen Komparator der ersten Signalverarbeitungsstufe der Pixelelemente der mehreren Pixelelemente umfassen.

Das Bereitstellen der Zählsignale kann ein Speichern auf einem computerlesbaren Speichermedium und/oder ein Anzeigen auf einer Darstellungseinheit und/oder ein Übertragen an eine Bereitstellungseinheit umfassen. Insbesondere kann eine graphische Darstellung der Zählsignale, insbesondere eine Rekonstruktion basierend auf den Zählsignalen, mittels der Darstellungseinheit angezeigbar sein.

In einer weiteren vorteilhaften Ausführungsform des vorgeschlagenen Verfahrens kann ein Röntgenbilddatensatz basierend auf den bereitgestellten Zählsignalen erzeugt werden.

Das Erzeugen des Röntgenbilddatensatzes kann eine Rekonstruktion von Bildwerten, insbesondere Voxelwerten oder Pixelwerten, basierend auf den Zählsignalen, insbesondere einer Anzahl der Zählsignale je Pixelelement, umfassen. Insbesondere können die Bildwerte des Röntgenbilddatensatzes basierend auf den Pixel-Zählsignalen, insbesondere einer Anzahl der Pixel-Zählsignale, rekonstruiert werden. Ferner können die jeweiligen Pixel-Zählsignale, insbesondere die jeweiligen Anzahlen der Pixel-Zählsignale, basierend auf den jeweiligen Koinzidenz-Zählsignalen, insbesondere jeweiligen Anzahlen der Koinzidenz-Zählsignale, korrigiert, insbesondere angepasst, werden. Beispielsweise kann pixelweise die jeweilige Anzahl von Koinzidenz-Zählsignalen von der jeweiligen Anzahl von Pixel-Zählsignalen subtrahiert werden. Beispielsweise kann eine gewichtete Subtraktion vorgesehen sein, insbesondere in dem vor der Subtraktion ein Beimischungsfaktor zu der Anzahl an Koinzidenz-Zählsignalen multipliziert wird. Das heißt, es kann lediglich ein Anteil oder ein Mehrfaches der zumindest einen Anzahl an Koinzidenz-Zählsignalen subtrahiert oder addiert werden.

In einer weiteren vorteilhaften Ausführungsform des vorgeschlagenen Verfahrens kann im Schritt c) überprüft werden, ob das Zählsignal ein Koinzidenz-Zählsignal aufweist. Verneinendenfalls können die definierte Pulslänge vergrößert und die Schritte a) bis c) wiederholt ausgeführt werden. Bejahendenfalls und bei keiner vorangegangenen Vergrößerung der definierten Pulslänge können die definierte Pulslänge verkleinert und die Schritte a) bis c) wiederholt ausgeführt werden. Andernfalls kann das Verfahren beendet werden.

Das Überprüfen, ob das Zählsignal ein Koinzidenz-Zählsignal aufweist, kann beispielsweise einen Vergleich eines Zählerstands des Koinzidenz-Zählelements vor Beginn des Verfahrens, insbesondere vor der momentanen Ausführung der Schritte a) bis c), mit einem momentanen Zählerstand des Koinzidenz-Zählelements umfassen. Eine Erhöhung des Zählerstands des Koinzidenz-Zählelements kann als Vorliegen eines Koinzidenz-Zählsignals identifiziert werden. Ein unveränderter Zählerstand des Koinzidenz-Zählelements kann als Nichtvorliegen eines Koinzidenz-Zählsignals identifiziert werden.

Verneinendenfalls, insbesondere bei Nichtvorliegen eines Koinzidenz-Zählsignals, kann die definierte Pulslänge vergrö-βert werden, beispielsweise um einen vorgegebenen Änderungswert. Ferner können verneinendenfalls die Schritte a) bis c) wiederholt ausgeführt werden.

Bejahendenfalls, insbesondere bei Vorliegen eines Koinzidenz-Zählsignals, kann ferner überprüft werden, ob die Schritte a) bis c) wiederholt ausgeführt worden sind und, ob die definierte Pulslänge bei der vorigen Ausführung der Schritte a) bis c) vergrößert worden ist. Bejahendenfalls, also bei Vorliegen eines Koinzidenz-Zählsignals und vorausgegangener Vergrößerung der definierten Pulslänge, kann das Verfahren beendet werden. Bei Vorliegen eines Koinzidenz-Zählsignals und keiner vorausgegangenen Vergrößerung der definierten Pulslänge können die definierte Pulslänge verkleinert werden, beispielsweise um den vorgegebenen Änderungswert oder einen weiteren vorgegebenen Änderungswert, und die Schritte a) bis c) wiederholt ausgeführt werden.

Vorteilhafterweise kann hierdurch die definierte Pulslänge optimiert, insbesondere minimiert, werden, wobei sichergestellt werden kann, dass Koinzidenz-Zählsignale mittels der optimierten definierten Pulslänge erfassbar sind. Die vorgeschlagene Ausführungsform kann als Tuning der definierten Pulslänge betrachtet werden derart, dass Koinzidenzen durch die jeweiligen Pulssignale verlässlich auslösbar sind. Hierdurch kann vorteilhaft eine Wahrscheinlichkeit von durch zu lange Pulslängen des jeweiligen Pulssignals fälschlicherweise ausgelösten Zusatzkoinzidenzen reduziert und somit eine Signalqualität optimiert werden.

Die Erfindung betrifft in einem dritten Aspekt ein Röntgengerät, umfassend eine Röntgenquelle und einen vorgeschlagenen photonenzählenden Röntgendetektor. Dabei ist die Röntgenquelle zum Aussenden von Röntgenstrahlung zur Beleuchtung des Röntgendetektors ausgebildet.

Die Vorteile des vorgeschlagenen Röntgengeräts entsprechen im Wesentlichen den Vorteilen des vorgeschlagenen photonenzählenden Röntgendetektors. Hierbei erwähnte Merkmale, Vorteile oder alternative Ausführungsformen können ebenso auch auf die anderen beanspruchten Gegenstände übertragen werden und umgekehrt.

Die Röntgenquelle und der Röntgendetektor können vorteilhafterweise in definierter Anordnung zueinander angeordnet sein. Ferner kann die definierte Anordnung von Röntgenquelle und Röntgendetektor beweglich, insbesondere rotierbar und/oder translatierbar, gelagert sein, beispielsweise bezüglich eines abzubildenden Untersuchungsobjekts. Die Röntgenquelle kann dazu ausgebildet sein, Röntgenstrahlung zur Beleuchtung des Röntgendetektors auszusenden. Der Röntgendetektor kann dazu ausgebildet sein, die eintreffende Röntgenstrahlung, beispielsweise nach einer Wechselwirkung mit dem Untersuchungsobjekt, mittels des Konverterelements in elektrische Signale umzuwandeln.

Vorteilhafterweise kann das Röntgengerät als Computertomographiegerät (CT-Gerät) und/oder C-Bogen-Röntgengerät und/oder O-Bogen-Röntgengerät ausgebildet sein.

Ausführungsbeispiele der Erfindung sind in den Zeichnungen dargestellt und werden im Folgenden näher beschrieben. In unterschiedlichen Figuren werden für gleiche Merkmale die gleichen Bezugszeichen verwendet. Es zeigen:
- Fig. 1 bis 6: schematische Darstellungen verschiedener vorteilhafter Ausführungsformen eines vorgeschlagenen photonenzählenden Röntgendetektors,
- Fig. 7 bis 9: schematische Darstellungen verschiedener vorteilhafter Ausführungsformen eines vorgeschlagenen Verfahrens zum Betreiben eines photonenzählenden Röntgengeräts,
- Fig. 10: eine schematische Darstellung eines CT-Geräts als beispielshafte Ausführungsform eines vorgeschlagenen Röntgengeräts.

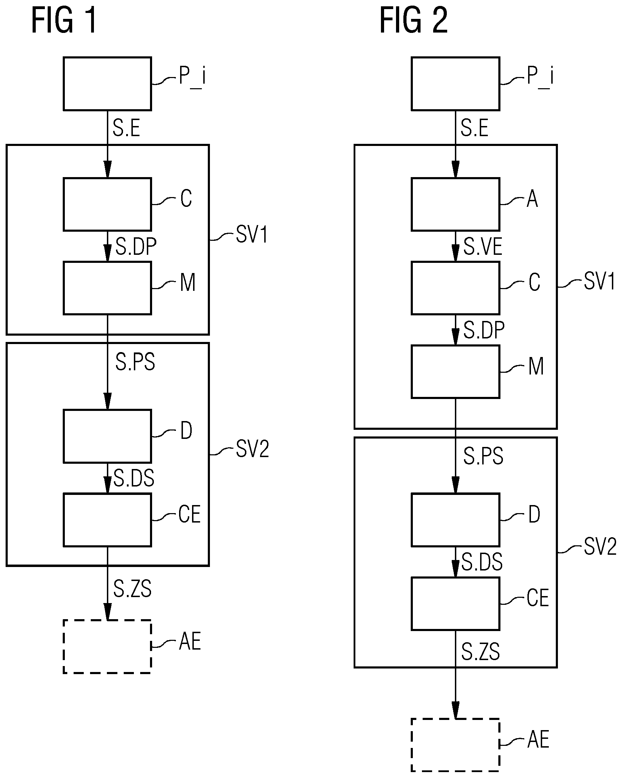

Fig. 1 zeigt eine schematische Darstellung einer vorteilhaften Ausführungsform eines vorgeschlagenen photonenzählenden Röntgendetektors. Der Röntgendetektor kann mehrere Pixelelemente und ein Konverterelement zur Umwandlung von Röntgenstrahlung in elektrische Signale S.E umfassen. In einer ersten Signalverarbeitungsstufe SV1 kann ein jeweiliges Pixelelement P_i der mehreren Pixelelemente jeweils zumindest einen Komparator C und die mehreren Pixelelemente zumindest eine Monoflop-Einheit M aufweisen. Der zumindest eine Komparator C kann dazu ausgebildet sein, das elektrische Signal S.E mit einem Signalschwellwert zu vergleichen und ein digitales Pixelsignal S.DP in Abhängigkeit des Vergleichs an die zumindest eine Monoflop-Einheit M bereitzustellen. Die zumindest eine Monoflop-Einheit M kann dazu ausgebildet sein, basierend auf dem digitalen Pixelsignal S.DP ein Pulssignal S.PS mit definierter Pulslänge an einen Ausgang der ersten Signalverarbeitungsstufe SV1 bereitzustellen. In einer zweiten Signalverarbeitungsstufe SV2 können die Ausgänge der ersten Signalverarbeitungsstufe der mehreren Pixelelemente, insbesondere des jeweiligen Pixelelements P_i, mit jeweils einer Verzögerungseinheit D signaltechnisch gekoppelt sein. Die jeweilige Verzögerungseinheit D kann dazu ausgebildet sein, die Pulssignale S.PS mit einer jeweils angepassten Verzögerung als angepasstes Pulssignal S.DS an jeweils ein Zählelement CE bereitzustellen. Dabei kann die jeweils angepasste Verzögerung auf einer schaltungsbedingten Verzögerung des jeweils zumindest einen Pixelelements P_i der mehreren Pixelelemente basieren. Das jeweilige Zählelement CE kann dazu ausgebildet sein, ein Zählsignal S.ZS in Abhängigkeit des jeweiligen zumindest einen angepassten Pulssignals S.DS zu zählen. Ferner kann das wenigstens eine Zählelement CE dazu ausgebildet sein, das Zählsignal S.ZS an eine Auswerteeinheit AE bereitzustellen. Die optionale Auswerteeinheit AE kann beispielsweise eine digitale Verarbeitungskette umfassen, insbesondere zur Berechnung, Verarbeitung und/oder Verwertung des jeweiligen Zählsignals S.ZS.

Vorteilhafterweise kann die zumindest eine Monoflop-Einheit M dazu ausgebildet sein, das Pulssignal S.PS bei Identifikation einer steigenden Flanke des digitalen Pixelsignals S.DP bereitzustellen. Vorteilhafterweise können die definierte Pulslänge des durch die zumindest eine Monoflop-Einheit M bereitstellbaren Pulssignals S.PS, insbesondere zwischen 1 ns und 20 ns, und/oder die Verzögerung des durch die zumindest eine Verzögerungseinheit D bereitstellbaren angepassten Pulssignals S.DS anpassbar sein.

Fig. 2 zeigt eine schematische Darstellung einer weiteren vorteilhaften Ausführungsform eines vorgeschlagenen photonenzählenden Röntgendetektors. Dabei können die Pixelelemente, insbesondere das jeweilige Pixelelement P_i der mehreren Pixelelemente, in der ersten Signalverarbeitungsstufe SV1 ferner jeweils einen Signalverstärker A aufweisen. Der Signalverstärker A kann dazu ausgebildet sein, das elektrische Signal S.E zu verstärken und das verstärkte elektrische Signal S.VE an den zumindest einen Komparator C bereitzustellen.

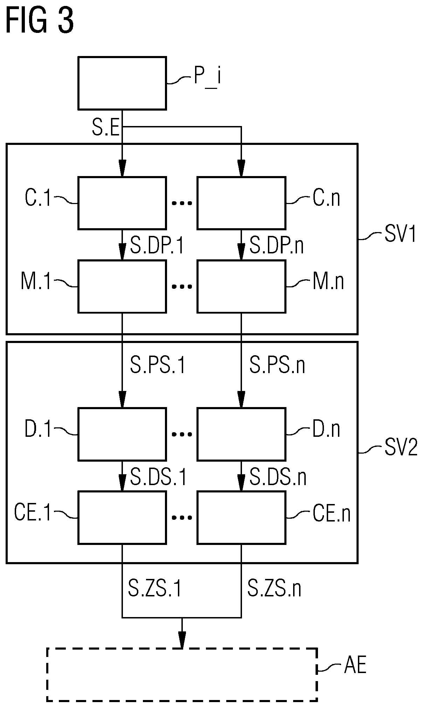

Fig. 3 zeigt eine schematische Darstellung einer weiteren vorteilhaften Ausführungsform eines vorgeschlagenen photonenzählenden Röntgendetektors. Dabei können die Pixelelemente, insbesondere das jeweilige Pixelelement P_i der mehreren Pixelelemente, in der ersten Signalverarbeitungsstufe SV1 jeweils mehrere Komparatoren C.1 bis C.n und zu wenigstens einem der mehreren Komparatoren C.1 bis C.n jeweils eine Monoflop-Einheit M.1 bis M.n aufweisen. Die Komparatoren C.1 bis C.n jeweils eines Pixelelements P_i der mehreren Pixelelemente können dazu ausgebildet sein, das elektrische Signal S.E mit verschiedenen Signalschwellwerten zu vergleichen und jeweils ein digitales Pixelsignal S.DP.1 bis S.DP.n in Abhängigkeit des Vergleichs an die jeweilige Monoflop-Einheit M.1 bis M.n bereitzustellen. Die Monoflop-Einheiten M.1 bis M.n können dazu ausgebildet sein, basierend auf dem jeweiligen digitalen Pixelsignal S.DP.1 bis S.DP.n jeweils ein Pulssignal S.PS.1 bis S.PS.n mit definierter Pulslänge an den jeweiligen Ausgang der ersten Signalverarbeitungsstufe SV1 bereitzustellen. Der Röntgendetektor kann in der zweiten Signalverarbeitungsstufe zu jedem Ausgang der ersten Signalverarbeitungsstufe, insbesondere zumindest zu jeder Monoflop-Einheit M.1 bis M.n, jeweils eine Verzögerungseinheit D.1 bis D.n aufweisen. Die jeweilige Verzögerungseinheit D.1 bis D.n kann dazu ausgebildet sein, das jeweilige Pulssignal S.PS.1 bis S.PS.n mit einer jeweils angepassten Verzögerung als angepasstes Pulssignal S.DS.1 bis S.DS.n an das jeweilige Zählelement CE.1 bis CE.n bereitzustellen.

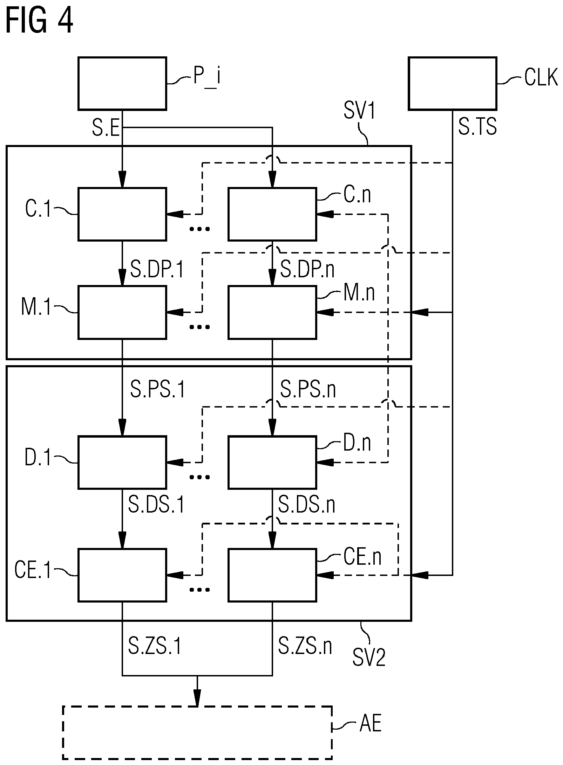

Fig. 4 zeigt eine schematische Darstellung einer weiteren vorteilhaften Ausführungsform eines vorgeschlagenen photonenzählenden Röntgendetektors. Dabei kann der Röntgendetektor ferner eine Taktsignalerzeugungseinheit CLK aufweisen, welche dazu ausgebildet ist, ein definiertes Taktsignal S.TS an die erste Signalverarbeitungsstufe SV1, insbesondere die mehreren Komparatoren C.1 bis C.n, die Monoflop-Einheiten M.1 bis M.n, und/oder die zweite Signalverarbeitungsstufe SV2, insbesondere die Verzögerungseinheiten D.1 bis D.n und/oder die Zähleinheiten CE.1 bis CE.n, bereitzustellen. Die erste Signalverarbeitungsstufe SV1 und/oder die zweite Signalverarbeitungsstufe SV2 können dazu ausgebildet sein, ihre jeweiligen Komponenten, welche zur zeitbasierten Verarbeitung und/oder Bereitstellung der jeweiligen Signale ausgebildet sind, insbesondere anhand des Taktsignals S.TS anzupassen, insbesondere die mehreren Komparatoren C.1 bis C.n, die Monoflop-Einheiten M.1 bis M.n, die Verzögerungseinheiten D.1 bis D.n, und/oder die Zähleinheiten CE.1 bis CE.n. Die Taktsignalerzeugungseinheit CLK kann mehrere Subtaktsignalerzeugungseinheiten aufweisen, welche zum Bereitstellen eines dedizierten Taktsignals für jeweils eine oder mehrere Komponenten des Röntgendetektors ausgebildet sein können.

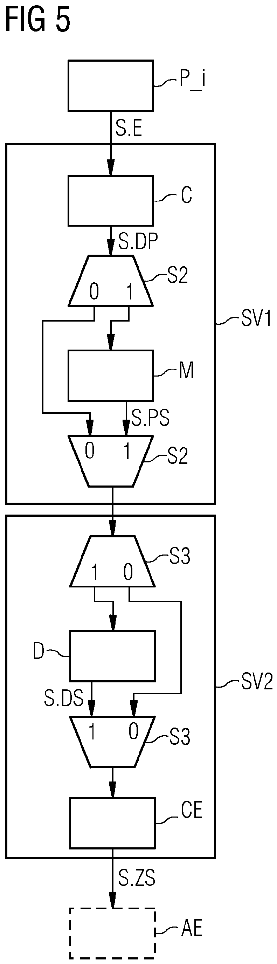

Fig. 5 zeigt eine schematische Darstellung einer weiteren vorteilhaften Ausführungsform eines vorgeschlagenen photonenzählenden Röntgendetektors. Dabei kann die erste Signalverarbeitungsstufe SV1 zumindest ein zweites Schaltelement S2 aufweist, welches dazu ausgebildet ist, die zumindest eine Monoflop-Einheit M zu aktivieren 1 oder zu deaktivieren 0. Das zweite Schaltelement kann einen Demultiplexer und einen Multiplexer umfassen. Dabei kann der zumindest eine Komparator C das digitale Pixelsignal S.DP bei deaktivierter 0 Monoflop-Einheit M an den jeweiligen Ausgang der ersten Signalverarbeitungsstufe SV1 bereitstellen.

Zudem kann die erste Signalverarbeitungsstufe SV1 zumindest ein drittes Schaltelement S3 aufweisen, welches dazu ausgebildet ist, die zumindest eine Verzögerungseinheit D zu aktivieren 1 oder zu deaktivieren 0. Das dritte Schaltelement kann einen Demultiplexer und einen Multiplexer umfassen. Bei Deaktivierung 0 der zumindest einen Verzögerungseinheit D kann das an die jeweilige Verzögerungseinheit D der zumindest einen Verzögerungseinheit D bereitgestellte Signal, insbesondere das Pulssignal S.PS oder das digitale Pixelsignal S.DP, verzögerungsfrei an das jeweilige Zählelement CE bereitstellbar sein.

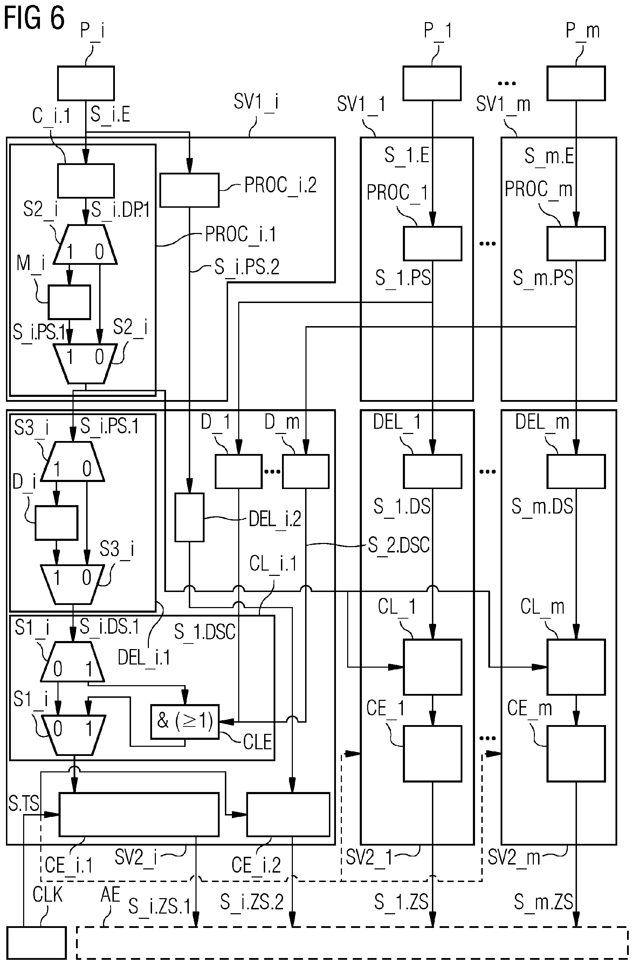

Fig. 6 zeigt eine schematische Darstellung einer weiteren vorteilhaften Ausführungsform eines vorgeschlagenen photonenzählenden Röntgendetektors. Die ersten Signalverarbeitungsstufen des jeweiligen Pixelelements P_i und weiterer Pixelelemente P_1 bis P_m sind entsprechend mit SV1_i, SV1_1 bis SV1_m und die zweiten Signalverarbeitungsstufen mit SV2_i, SV2_1 bis SV2_m bezeichnet. Die jeweiligen Verarbeitungsketten zur Verarbeitung der jeweiligen elektrischen Signale S_i.E, S_1.E bis S_m.E in der jeweiligen ersten Signalverarbeitungsstufe SV1_i, SV1_1 bis SV1_m sind mit PROC_i.1 und PROC_i.2 für das jeweilige Pixelelement P_i und mit PROC_1 bis PROC_m für die weiteren Pixelelemente P_1 bis P_m bezeichnet. Die Verarbeitungsketten PROC_i.1, PROC_i.2 und PROC_1 bis PROC_m können im Wesentlichen analog ausgebildet sein. Die an den Ausgängen der jeweiligen ersten Signalverarbeitungsstufen bereitstellbaren Pulssignale S_i.PS_1 und S_i.PS_2 des jeweiligen Pixelelements P_i und S_1.PS bis S_m.PS der weiteren Pixelelemente P_1 bis P_m sind an jeweilige zweite Verarbeitungsketten DEL_i.1, DEL_i.2 und DEL_1 bis DEL_m zur Verzögerung der jeweiligen Signale in der jeweiligen zweiten Signalverarbeitungsstufe SV2_i und SV2_1 bis SV2_m bezeichnet. Die Verarbeitungsketten DEL_i.1, DEL_i.2 und DEL_1 bis DEL_m können im Wesentlichen analog ausgebildet sein. Ferner werden die Zählelemente der jeweiligen Pixelelemente entsprechend mit CE_i.1 und CE_i.2 für das jeweilige Pixelelement P_i und mit CE_1 bis CE_m für die weiteren Pixelelemente P_1 bis P_m bezeichnet. Die jeweiligen Zählsignale sind mit S_i.ZS.1 und S_i.ZS.2 für das jeweilige Pixelelement P_i und mit S_1.ZS bis S_m.ZS für die weiteren Pixelelemente P_1 bis P_m bezeichnet.

In der zweiten Signalverarbeitungsstufe SV2_i des jeweiligen Pixels P_i können die Verzögerungseinheiten zumindest einer Gruppe von Pixelelementen P_i, P_1 bis P_m der mehreren Pixelelemente, insbesondere die zumindest eine Verzögerungseinheit D_i des jeweiligen Pixelelements P_i und Verzögerungseinheiten D_1 bis D_m der weiteren Pixelelemente P_1 bis P_m, mit einer Koinzidenzlogik CLE und jeweils einem Zählelement CE_i.1 signaltechnisch gekoppelt sein. Die weiteren Pixelelemente P_1 bis Pm können analog zu dem jeweiligen Pixelelement P_i in ihrer jeweiligen zweiten Signalverarbeitungsstufe SV2_1 bis SV2_m jeweilige Verzögerungsketten DEL_1 bis DEL_m aufweisen. Die in der zweiten Signalverarbeitungsstufe SV2_i des jeweiligen Pixelelements P_i umfassten Verzögerungseinheiten D_1 bis D_m können verschieden oder gleich zu den in den jeweiligen Verzögerungsketten DEL_1 bis DEL_m umfassten Verzögerungseinheiten sein.

Die, insbesondere angepassten Pulssignale, S_i.DS.1 und S_1.DSC bis S_m.DSC der zumindest einen Gruppe von Pixelelementen P_i und P_1 bis P_m können an die Koinzidenzlogik CLE bereitstellbar sein. Die Koinzidenzlogik CLE kann dazu ausgebildet sein, ein koinzidierendes Auftreten von angepassten Pulssignalen S_i.DS.1 des jeweiligen Pixelelements P_i und angepassten Pulssignalen S_1.DSC bis S_m.DSC der weiteren Pixelelemente P_1 bis P_m der zumindest einen Gruppe von Pixelelementen zu identifizieren. Das zumindest eine Zählelement CE_i.1 kann dazu ausgebildet sein, bei Identifikation einer Koinzidenz durch die Koinzidenzlogik CLE ein Koinzidenz-Zählsignal zu zählen, welches auf dem in dem jeweiligen Pixelelement P_i direkt eingegangenen Signal, insbesondere oberhalb des Signalschwellwerts des Komparators C_i.1, und auf dem koinzidierend auftretenden angepassten Pulssignal S_1.DSC bis S_m.DSC der weiteren Pixelelemente P_1 bis P_m der mehreren Pixelelemente basiert. Die Pulssignale S_1.PS bis S_m.PS der weiteren Pixelelemente können mittels weiterer Verzögerungseinheiten D_1 bis D_m mit einer jeweils angepassten Verzögerung an die Koinzidenzlogik als die jeweils angepassten Pulssignale S_1.DSC bis S_m.DSC bereitstellbar sein. Ferner können die weiteren Pixelelemente P_1 bis P_m analoge erste Signalverarbeitungsstufen SV1_1 bis SV1_m und analoge zweite Signalverarbeitungsstufen SV2_1 bis SV2_m wie das jeweilige Pixelelement P_i aufweisen. Das zumindest eine Zählelement CE_i.1 kann dazu ausgebildet sein, das jeweilige Zählsignal S_i.ZS.1 basierend auf dem ersten Pixel-Zählsignal und dem Koinzidenz-Zählsignal bereitzustellen.

Vorteilhafterweise kann die zweite Signalverarbeitungsstufe SV2_i des jeweiligen Pixelelements P_i zumindest ein erstes Schaltelement S1_i aufweisen, welches dazu ausgebildet ist, die Koinzidenzlogik CL_i zu aktivieren 1 oder zu deaktivieren 0. Das erste Schaltelement kann einen Demultiplexer und einen Multiplexer umfassen. Die Zählelemente CE_i.1 der zumindest einen Gruppe von Pixelelementen können bei deaktivierter 0 Koinzidenzlogik CLE das Pixel-Zählsignal zählen.

Die jeweiligen Verarbeitungsketten zur Verarbeitung der angepassten Pulssignale S_1.DS bis S_m.DS der zumindest einen Gruppe von Pixelelementen, jeweils umfassend eine Koinzidenzlogik CLE und ein erstes Schaltelement, werden in Fig. 6 entsprechend als CL_i.1 und CL_1 bis CL_m bezeichnet.

Das jeweilige Pixelelement P_i kann in der ersten Signalverarbeitungsstufe SV1_i ferner wenigstens eine weitere Verarbeitungskette PROC_i.2 umfassen, welche, insbesondere hinsichtlich des Signalschwellwerts des jeweiligen Komparators und/oder der jeweiligen Monoflop-Einheit, verschieden von der ersten Verarbeitungskette PROC_i.1 des jeweiligen Pixelelements P_i sein kann.

Vorteilhafterweise kann die zumindest eine Gruppe von Pixelelementen zumindest eines der Pixelelemente P_i und zumindest ein weiteres an das Pixelelement P_i reihen- oder spaltenweise direkt angrenzendes Pixelelement P_1 bis P_m umfassen.

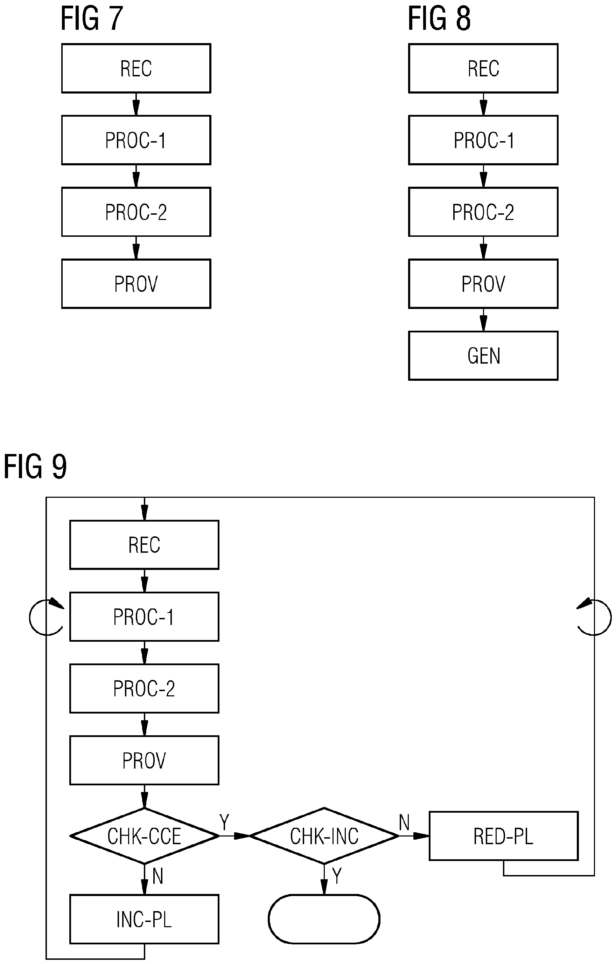

Fig. 7 zeigt eine schematische Darstellung einer vorteilhaften Ausführungsform eines vorgeschlagenen Verfahrens zum Betreiben eines photonenzählenden Röntgengeräts. In einem ersten Schritt a) kann Röntgenstrahlung mit dem photonenzählenden Röntgendetektor empfangen werden REC. Dabei kann die Röntgenstrahlung mittels des Konverterelements in elektrische Signale S.E umgewandelt werden. In einem weiteren Schritt b) können die erzeugten elektrischen Signale S.E mittels der ersten Signalverarbeitungsstufe SV1 und der zweiten Signalverarbeitungsstufe SV2 des photonenzählenden Röntgendetektors verarbeitet werden PROC-1. Dabei können die Pulssignale S.PS an die Ausgänge der ersten Signalverarbeitungsstufe SV1 bereitgestellt werden, welche mittels der zweiten Signalverarbeitungsstufe SV2 weiterverarbeitet werden PROC-2. In einem weiteren Schritt c) können die Zählsignale S.ZS der Zählelemente CE der zweiten Signalverarbeitungsstufe SV2 bereitgestellt werden PROV.

Fig. 8 zeigt eine schematische Darstellung einer weiteren vorteilhaften Ausführungsform eines vorgeschlagenen Verfahrens zum Betreiben eines photonenzählenden Röntgengeräts. Dabei kann ein Röntgenbilddatensatz basierend auf den bereitgestellten Zählsignalen S.ZS erzeugt werden GEN.

Fig. 9 zeigt eine schematische Darstellung einer weiteren vorteilhaften Ausführungsform eines vorgeschlagenen Verfahrens zum Betreiben eines photonenzählenden Röntgengeräts. Dabei kann im Schritt c) überprüft werden CHK-CCE, ob das Zählsignal S.ZS ein Koinzidenz-Zählsignal aufweist. Verneinendenfalls N können die definierte Pulslänge vergrößert INC-PL und die Schritte a) bis c) wiederholt ausgeführt werden. Bejahendenfalls Y kann überprüft werden CHK-INK, ob es eine vorausgegangene Ausführung der Schritte a) bis c) mit einer Vergrö-βerung der definierten Pulslänge INC-PL gab. Verneinendenfalls N, also bei keiner vorangegangenen Vergrößerung der definierten Pulslänge INC-PL, können die definierte Pulslänge verkleinert RED-PL und die Schritte a) bis c) wiederholt ausgeführt werden. Andernfalls Y kann das Verfahren beendet werden.

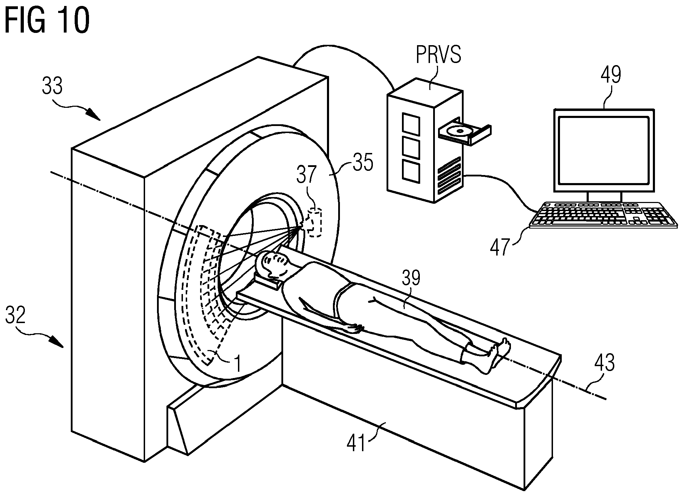

Fig. 10 zeigt eine schematische Darstellung einer vorteilhaften Ausführungsform eines vorgeschlagenen Röntgengeräts als medizinisches CT-Gerät 33. Das CT-Gerät 33 kann die Röntgenquelle 37, einen vorgeschlagenen photonenzählenden Röntgendetektor 1 und eine Verarbeitungseinheit PRVS umfassen. Dabei können die Röntgenquelle 37 und der Röntgendetektor 1 in Gegenüberstellung zueinander angeordnet sein. Die Röntgenquelle 37 kann dazu ausgebildet sein, den Röntgendetektor 1 entlang einer Röntgenstrahleneinfallsrichtung mit Röntgenstrahlung zu beleuchten. Der Röntgendetektor 1 kann eine direkt-konvertierende (Halbleiter-)Röntgendetektorschicht umfassen. Dabei kann die Röntgendetektorschicht beispielsweise CdTe, CdZnTe, CdTeSe, CdZnTeSe oder CdMnTe als Halbleitermaterial aufweisen.

Das CT-Gerät 33 kann zudem eine Gantry 32 mit einem Rotor 35 umfassen. Die Röntgenquelle 37 und der Röntgendetektor 1 können in definierter Anordnung an dem Rotor 35 angeordnet sein, insbesondere in den Rotor 35 integriert oder an dem Rotor 35 befestigt sein. Der Rotor 35 kann um eine Rotationsachse 43 drehbar gelagert sein. Das abzubildendes Untersuchungsobjekt 39 kann auf der Patientenlagerungsvorrichtung 41 gelagert und entlang der Rotationsachse 43 durch die Gantry 32 hindurch bewegbar sein. Zur Steuerung des CT-Geräts 33 und zur Berechnung von Schnittbildern bzw. Volumenbildern des Untersuchungsobjekts 39 kann die Verarbeitungseinheit PRVS verwendet werden. Eine Eingabeeinrichtung 47, beispielsweise eine Tastatur, und eine Ausgabevorrichtung 49, beispielsweise ein Bildschirm und/oder Display, können mit der Verarbeitungseinheit PRVS verbunden, insbesondere signaltechnisch gekoppelt, sein. Die Eingabeeinrichtung 47 kann vorteilhafterweise in die Ausgabevorrichtung 49 integriert sein, beispielsweise bei einem, insbesondere resistiven und/oder kapazitiven, Eingabedisplay. Die Ausgabevorrichtung 49 kann zur Anzeige einer graphischen Darstellung der Zählsignale und/oder des Röntgenbilddatensatzes ausgebildet sein.

Die in den beschriebenen Figuren enthaltenen schematischen Darstellungen bilden keinerlei Maßstab oder Größenverhältnisse ab.

Es wird abschließend noch einmal darauf hingewiesen, dass es sich bei den vorhergehend detailliert beschriebenen Verfahren sowie bei den dargestellten Vorrichtungen lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriffe "Einheit" und "Element" nicht aus, dass die betreffenden Komponenten aus mehreren zusammenwirkenden Teil-Komponenten bestehen, die gegebenenfalls auch räumlich verteilt sein können.

Der Ausdruck "basierend auf "kann im Kontext der vorliegenden Anmeldung insbesondere im Sinne des Ausdrucks "unter Verwendung von" verstanden werden. Insbesondere schließt eine Formulierung, derzufolge ein erstes Merkmal basierend auf einem zweiten Merkmal erzeugt (alternativ: ermittelt, bestimmt etc.) wird, nicht aus, dass das erste Merkmal basierend auf einem dritten Merkmal erzeugt (alternativ: ermittelt, bestimmt etc.) werden kann.

## Patentansprüche

1. Photonenzählender Röntgendetektor (1), umfassend mehrere Pixelelemente (P_i, P_1, P_m) und ein Konverterelement zur Umwandlung von Röntgenstrahlung in elektrische Signale (S.E, S_i.E, S_1.E, S_m.E),
wobei in einer ersten Signalverarbeitungsstufe (SV1, SV1_i, SV1_1, SV1_m) die Pixelelemente (P_i) der mehreren Pixelelemente (P_i, P_1, P_m) jeweils zumindest einen Komparator (C, C_i, C.1, C.n, C_i.1, C_i.2) und die mehreren Pixelelemente (P_i, P_1, P_m) zumindest eine Monoflop-Einheit (M, M_i, M.1, M.n) aufweisen,
wobei der zumindest eine Komparator (C, C_i, C.1, C.n, C_i.1, C_i.2) dazu ausgebildet ist, das elektrische Signal (S.E, S_i.E, S_1.E, S_m.E) mit einem Signalschwellwert zu vergleichen und ein digitales Pixelsignal (S.DP, S.DP.1, S.DP.n, S_i.DP) in Abhängigkeit des Vergleichs an die zumindest eine Monoflop-Einheit (M, M_i, M.1, M.n) bereitzustellen,
wobei die zumindest eine Monoflop-Einheit (M, M_i, M.1, M.n) dazu ausgebildet ist, basierend auf dem digitalen Pixelsignal (S.DP, S.DP.1, S.DP.n, S_i.DP) ein Pulssignal (S.PS, S.PS.1, S.PS.n, S_i.PS.1, S_i.PS.2, S_1.PS, S_m.PS) mit definierter Pulslänge an einen Ausgang der ersten Signalverarbeitungsstufe (SV1, SV1_i, SV1_1, SV1_m) bereitzustellen,
wobei in einer zweiten Signalverarbeitungsstufe (SV2, SV2_i, SV2_1, SV2_m) die Ausgänge der ersten Signalverarbeitungsstufe (SV1, SV1_i, SV1_1, SV1_m) der mehreren Pixelelemente (P_i, P_1, P_m) mit jeweils einer Verzögerungseinheit (D, D_i, D.1, D.n) signaltechnisch gekoppelt sind,
wobei die jeweilige Verzögerungseinheit (D, D_i, D.1, D.n) dazu ausgebildet ist, die Pulssignale (S.PS, S.PS.1, S.PS.n, S_i.PS.1, S_i.PS.2, S_1.PS, S_m.PS) mit einer jeweils angepassten Verzögerung als angepasste Pulssignale (S.DS, S.DS.1, S.DS.n, S_i.DS.1, S_i.DS.2, S_1.DS, S_m.DS) an jeweils ein Zählelement (CE, CE.1, CE.n, CE_i.1, CE_i.2) bereitzustellen, wobei die jeweils angepasste Verzögerung auf einer schaltungsbedingten Verzögerung des jeweils zumindest einen Pixelelements (P_i) der mehreren Pixelelemente (P_i, P_1, P_m) basiert,
wobei das jeweilige Zählelement (CE, CE.1, CE.n, CE_i.1, CE_i.2) dazu ausgebildet ist, ein Zählsignal (S.ZS, S.ZS.1, S.ZS.n, S_i.ZS.1, S_i.ZS.2, S_1.ZS, S_m.ZS) in Abhängigkeit des jeweiligen zumindest einen angepassten Pulssignals (S.DS, S.DS.1, S.DS.n, S_i.DS.1, S_i.DS.2, S_1.DS, S_m.DS) zu zählen.

2. Photonenzählender Röntgendetektor (1) nach Anspruch 1, wobei die Pixelelemente in der ersten Signalverarbeitungsstufe (SV1, SV1_i, SV1_1, SV1_m) ferner jeweils einen Signalverstärker (A) aufweisen,
wobei der Signalverstärker (A) dazu ausgebildet ist, das elektrische Signal (S.E, S_i.E, S_1.E, S_m.E) zu verstärken und das verstärkte elektrische Signal (S.VE) an den zumindest einen Komparator (C, C_i, C.1, C.n, C_i.1, C_i.2) bereitzustellen.

3. Photonenzählender Röntgendetektor (1) nach Anspruch 1 oder 2,
wobei die zumindest eine Monoflop-Einheit (M, M_i, M.1, M.n) dazu ausgebildet ist, das Pulssignal (S.PS, S.PS.1, S.PS.n, S_i.PS.1, S_i.PS.2, S_1.PS, S_m.PS) bei Identifikation einer steigenden Flanke des digitalen Pixelsignals (S.DP, S.DP.1, S.DP.n, S_i.DP) bereitzustellen.

4. Photonenzählender Röntgendetektor (1) nach einem der vorangehenden Ansprüche,
wobei die Pixelelemente (P_i) in der ersten Signalverarbeitungsstufe (SV1, SV1_i, SV1_1, SV1_m) jeweils mehrere Komparatoren (C, C_i, C.1, C.n, C_i.1, C_i.2) und zu jedem Komparator (C, C_i, C.1, C.n, C_i.1, C_i.2) jeweils eine Monoflop-Einheit (M, M_i, M.1, M.n) aufweisen,
wobei die Komparatoren (C, C_i, C.1, C.n, C_i.1, C_i.2) jeweils eines der mehreren Pixelelemente (P_i, P_1, P_m) dazu ausgebildet sind, das elektrische Signal (S.E, S_i.E, S_1.E, S_m.E) mit verschiedenen Signalschwellwerten zu vergleichen und jeweils ein digitales Pixelsignal (S.DP, S.DP.1, S.DP.n, S_i.DP) in Abhängigkeit des Vergleichs an die jeweilige Monoflop-Einheit (M, M_i, M.1, M.n) bereitzustellen,
wobei die Monoflop-Einheiten (M, M_i, M.1, M.n) dazu ausgebildet sind, basierend auf dem jeweiligen digitalen Pixelsignal (S.DP, S.DP.1, S.DP.n, S_i.DP) jeweils ein Pulssignal (S.PS, S.PS.1, S.PS.n, S_i.PS.1, S_i.PS.2, S_1.PS, S_m.PS) mit definierter Pulslänge an den jeweiligen Ausgang der ersten Signalverarbeitungsstufe (SV1, SV1_i, SV1_1, SV1_m) bereitzustellen.

5. Photonenzählender Röntgendetektor (1) nach einem der vorangehenden Ansprüche,
wobei in der zweiten Signalverarbeitungsstufe (SV2, SV2_i, SV2_1, SV2_m) die Verzögerungseinheiten (D, D_i, D.1, D.n) zumindest einer Gruppe von Pixelelementen (P_i, P_1, P_m) der mehreren Pixelelemente (P_i, P_1, P_m) mit einer Koinzidenzlogik (CLE) und jeweils einem Zählelement (CE, CE.1, CE.n, CE_i.1, CE_i.2) signaltechnisch gekoppelt sind,
wobei die angepassten Pulssignale (S.DS, S.DS.1, S.DS.n, S_i.DS.1, S_i.DS.2, S_1.DS, S_m.DS) der zumindest einen Gruppe von Pixelelementen (P_i, P_1, P_m) an die Koinzidenzlogik (CLE) bereitstellbar sind,
wobei die Koinzidenzlogik (CLE) dazu ausgebildet ist, ein koinzidierendes Auftreten von angepassten Pulssignalen (S.DS, S.DS.1, S.DS.n, S_i.DS.1, S_i.DS.2, S_1.DS, S_m.DS) mehrerer Pixelelemente (P_i, P_1, P_m) zu identifizieren,
wobei die jeweiligen Zählelemente (CE, CE.1, CE.n, CE_i.1, CE_i.2) der zumindest einen Gruppe von Pixelelementen (P_i, P_1, P_m) dazu ausgebildet sind:
- ein Pixel-Zählsignal zu zählen, welches auf einem in den Pixelelementen (P_i, P_1, P_m) der Gruppe von Pixelelementen (P_i, P_1, P_m) direkt eingegangenen Signal basiert oder
- bei Identifikation einer Koinzidenz durch die Koinzidenzlogik (CLE) ein Koinzidenz-Zählsignal zu zählen, welches auf dem in dem jeweiligen Pixelelement (P_i) direkt eingegangenen Signal und auf dem koinzidierend auftretenden Signal zumindest eines weiteren Pixelelements (P_1, P_m) der mehreren Pixelelemente (P_i, P_1, P_m) basiert.

6. Photonenzählender Röntgendetektor (1) nach Anspruch 5, wobei die zweite Signalverarbeitungsstufe (SV2, SV2_i, SV2_1, SV2_m) zumindest ein erstes Schaltelement (S1_i) aufweist, welches dazu ausgebildet ist, die Koinzidenzlogik (CLE) zu aktivieren oder zu deaktivieren,
wobei die Zählelemente (CE, CE.1, CE.n, CE_i.1, CE_i.2) der zumindest einen Gruppe von Pixelelementen (P_i, P_1, P_m) bei deaktivierter Koinzidenzlogik (CLE) das Pixel-Zählsignal zählen.

7. Photonenzählender Röntgendetektor (1) nach Anspruch 5 oder 6,
wobei die zumindest eine Gruppe von Pixelelementen (P_i, P_1, P_m) zumindest eines der Pixelelemente (P_i) und zumindest ein weiteres an das Pixelelement (P_i) reihen- oder spaltenweise direkt angrenzendes Pixelelement (P_1, P_m) umfasst.

8. Photonenzählender Röntgendetektor (1) nach einem der vorangehenden Ansprüche,
weiterhin aufweisend eine Taktsignalerzeugungseinheit (CLK), welche dazu ausgebildet ist, ein definiertes Taktsignal (S.TS) an die erste und/oder zweite Signalverarbeitungsstufe (SV1, SV1_i, SV1_1, SV1_m, SV2, SV2_i, SV2_1, SV2_m) bereitzustellen,
wobei die erste und/oder die zweite Signalverarbeitungsstufe (SV1, SV1_i, SV1_1, SV1_m, SV2, SV2_i, SV2_1, SV2_m) dazu ausgebildet sind, ihre jeweiligen Komponenten, welche zur zeitbasierten Verarbeitung und/oder Bereitstellung der jeweiligen Signale ausgebildet sind, anhand des Taktsignals (S.TS) anzupassen.

9. Photonenzählender Röntgendetektor (1) nach einem der vorangehenden Ansprüche,
wobei die erste Signalverarbeitungsstufe (SV1, SV1_i, SV1_1, SV1_m) zumindest ein zweites Schaltelement (S2, S2_i) aufweist, welches dazu ausgebildet ist, die zumindest eine Monoflop-Einheit (M, M_i, M.1, M.n) zu aktivieren oder zu deaktivieren,
wobei der zumindest eine Komparator (C, C_i, C.1, C.n, C_i.1, C_i.2) das digitale Pixelsignal (S.DP, S.DP.1, S.DP.n, S_i.DP) bei deaktivierter Monoflop-Einheit (M, M_i, M.1, M.n) an den jeweiligen Ausgang der ersten Signalverarbeitungsstufe (SV1, SV1_i, SV1_1, SV1_m) bereitstellt.

10. Photonenzählender Röntgendetektor (1) nach einem der vorangehenden Ansprüche,
wobei die zweite Signalverarbeitungsstufe (SV2, SV2_i, SV2_1, SV2_m) zumindest ein drittes Schaltelement (S3, S3_i) aufweist, welches dazu ausgebildet ist, die zumindest eine Verzögerungseinheit (D, D_i, D.1, D.n) zu aktivieren oder zu deaktivieren,
wobei bei Deaktivierung der zumindest einen Verzögerungseinheit (D, D_i, D.1, D.n) das an die jeweilige Verzögerungseinheit (D, D_i, D.1, D.n) der zumindest einen Verzögerungseinheit (D, D_i, D.1, D.n) bereitgestellte Signal verzögerungsfrei an das jeweilige Zählelement (CE, CE.1, CE.n, CE_i.1, CE_i.2) bereitstellbar ist.

11. Photonenzählender Röntgendetektor (1) nach einem der vorangehenden Ansprüche,
wobei die definierte Pulslänge des durch die zumindest eine Monoflop-Einheit (M, M_i, M.1, M.n) bereitstellbaren Pulssignals (S.PS, S.PS.1, S.PS.n, S_i.PS.1, S_i.PS.2, S_1.PS, S_m.PS), insbesondere zwischen 1 ns und 20 ns, und/oder die Verzögerung des durch die zumindest eine Verzögerungseinheit (D, D_i, D.1, D.n) bereitstellbaren angepassten Pulssignals (S.DS, S.DS.1, S.DS.n, S_i.DS.1, S_i.DS.2, S_1.DS, S_m.DS) anpassbar sind.

12. Verfahren zum Betreiben eines photonenzählenden Röntgendetektors (1), wobei der Röntgendetektor (1) nach einem der vorangehenden Ansprüche ausgebildet ist, umfassend:
a) Empfangen (REC) von Röntgenstrahlung mit dem photonenzählenden Röntgendetektor (1), wobei die Röntgenstrahlung mittels des Konverterelements in elektrische Signale (S.E, S_i.E, S_1.E, S_m.E) umgewandelt wird,
b) Verarbeiten (PROC-1, PROC-2) der erzeugten elektrischen Signale (S.E, S_i.E, S_1.E, S_m.E) mittels der ersten Signalverarbeitungsstufe (SV1, SV1_i, SV1_1, SV1_m) und der zweiten Signalverarbeitungsstufe (SV2, SV2_i, SV2_1, SV2_m) des photonenzählenden Röntgendetektors (1),
wobei Pulssignale (S.PS, S.PS.1, S.PS.n, S_i.PS.1, S_i.PS.2, S_1.PS, S_m.PS) an die Ausgänge der ersten Signalverarbeitungsstufe (SV1, SV1_i, SV1_1, SV1_m) bereitgestellt werden, welche mittels der zweiten Signalverarbeitungsstufe (SV2, SV2_i, SV2_1, SV2_m) weiterverarbeitet werden,
c) Bereitstellen (PROV) der Zählsignale (S.ZS, S.ZS.1, S.ZS.n, S_i.ZS.1, S_i.ZS.2, S_1.ZS, S_m.ZS) der Zählelemente (CE, CE.1, CE.n, CE_i.1, CE_i.2) der zweiten Signalverarbeitungsstufe (SV2, SV2_i, SV2_1, SV2_m).

13. Verfahren nach Anspruch 12,
wobei ein Röntgenbilddatensatz basierend auf den bereitgestellten Zählsignalen (S.ZS, S.ZS.1, S.ZS.n, S_i.ZS.1, S_i.ZS.2, S_1.ZS, S_m.ZS) erzeugt wird (GEN).

14. Verfahren nach Anspruch 12,
wobei der Röntgendetektor (1) nach einem der Ansprüche 5 bis 7 und Anspruch 11 ausgebildet ist,
wobei im Schritt c) überprüft wird (CHCK-CCE), ob das Zählsignal (S.ZS, S.ZS.1, S.ZS.n, S_i.ZS.1, S_i.ZS.2, S_1.ZS, S_m.ZS) ein Koinzidenz-Zählsignal aufweist,
wobei verneinendenfalls die definierte Pulslänge vergrößert (INC-PL) und die Schritte a) bis c) wiederholt ausgeführt werden,
wobei bejahendenfalls und bei keiner vorangegangenen Vergrö-βerung der definierten Pulslänge die definierte Pulslänge verkleinert (RED-PL) und die Schritte a) bis c) wiederholt ausgeführt werden,
wobei das Verfahren andernfalls beendet wird.

15. Röntgengerät, umfassend eine Röntgenquelle (37) und einen photonenzählenden Röntgendetektor (1) nach einem der Ansprüche 1 bis 11,
wobei die Röntgenquelle (37) zum Aussenden von Röntgenstrahlung zur Beleuchtung des Röntgendetektors (1) ausgebildet ist.
